# EUROPEAN PATENT SPECIFICATION

(11) **EP 2 081 553 B1**
(45) Date of publication and mention of the grant of the patent: **12.08.2020**
(21) Application number: 07839335.2
(22) Date of filing: 05.10.2007
(51) Int. Cl.: A61K 9/19, A61K 47/02, A61K 47/26, A61K 9/00, A61K 39/395

(54) **STABLE ANTIBODY FORMULATIONS**
STABILE ANTIKÖRPER-FORMULIERUNGEN
FORMULATIONS STABLES COMPRENANT DES ANTICORPS

(30) Priority: 06.10.2006 US 850362 P; 10.10.2006 US 850970 P
(43) Date of publication of application: 29.07.2009
(73) Proprietor: Amgen Inc., Thousand Oaks, CA 91320-1799 (US)
(72) Inventor: MCAULEY, Arnold, Moorpark, CA 93021 (US); REHDER, Douglas, Woodland Hills, CA 91364 (US); MATSUMURA, Masazumi, Thousand Oaks, CA 91362 (US)
(74) Representative: Grünecker Patent- und Rechtsanwälte PartG mbB
(86) International application number: PCT/US2007/021477
(87) International publication number: WO 2008/045373

(56) References cited:
- WO-A-03/039485
- WO-A-2004/039826
- WO-A-2006/096461
- WO-A-2006/107854
- WO-A-2007/145862
- WO-A2-02/096457
- US-A1- 2004 191 243
- Anonymous: "Buffer Solutions", How do I Solve It ?, 5 April 2016 (2016-04-05), pages 1/5-5/5, XP055262797, Retrieved from the Internet: URL:https://www.chem.purdue.edu/gchelp/how tosolveit/Equilibrium/Buffers.htm [retrieved on 2016-04-05]

## Description

### BACKGROUND OF THE INVENTION

This invention relates generally to medicines for the treatment of diseases and, more specifically to consistently stable formulations for therapeutic molecules.

With the advent of recombinant DNA technology, protein-based therapeutics have become continually and increasingly commonplace in the repertoire of drugs available to medical practitioners for the treatment of a wide range of diseases from cancer to autoimmune diseases. Along with the scientific and technical advances that have occurred in the production of recombinant proteins, another reason for the success of protein therapeutics is their high specificity towards targets and their ability to exhibit superior safety profiles when compared to small molecule therapeutics. The ability to employ biological molecules as pharmaceuticals in the treatment of diseases has significantly advanced medical care and quality of life over the past quarter of a century.

Proteins known to exhibit various pharmacological actions *in vivo* are now capable of being produced in large amounts for various pharmaceutical applications. Long-term stability of a therapeutic protein is a particularly beneficial criterion for safe, consistent and efficacious treatments. Loss of functionality of the therapeutic within a preparation will decrease its effective concentration for a given administration. Similarly, undesired modifications of a therapeutic can affect the activity and/or the safety of a preparation, leading to loss of efficacy and risk of adverse side effects.

Proteins are complex molecules with defined primary, secondary, tertiary and in some cases quaternary structures, all of which play a role in imparting specific biological function. Structural complexity of biological pharmaceuticals such as proteins make them susceptible to various processes that result in structural and functional instability as well as loss of safety. With respect to these instability processes or degradation pathways, a protein can undergo a variety of covalent and non-covalent reactions or modifications in solution. For example, protein degradation pathways can be generally classified into two main categories: (i) physical degradation or non-covalent pathways, and (ii) chemical or covalent degradation pathways.

Protein drugs are susceptible to the physical degradation process of irreversible aggregation. Protein aggregation is of particular interest in polypeptide production because it often results in diminished bioactivity that affects drug potency, and also can elicit serious immunological or antigenic reactions in patients. Chemical degradation of a protein therapeutic, including degradation of the chemical structure by, for example, chemical modification, also has been implicated in increasing its immunogenic potential. Thus, stable protein formulations require that both physical and chemical degradation pathways of the drug be minimized.

Proteins can degrade, for example, via physical processes such as interfacial adsorption and aggregation. Adsorption can significantly impact a protein drug's potency and stability. It can cause an appreciable loss in potency of low concentration dosage forms. A second consequence is that unfolding mediated adsorption at interfaces can often be an initiating step for irreversible aggregation in solution. In this respect, proteins tend to adsorb at liquid-solid, liquid-air, and liquid-liquid interfaces. Sufficient exposure of a protein's core at a hydrophobic surface can result in adsorption as a consequence of agitation, temperature or pH induced stresses. Further, proteins also are sensitive to, for example, pH, ionic strength, thermal stress, shear and interfacial stresses, all of which can lead to aggregation and result in instability. Another consequence of aggregation is particle formation an important consideration in liquid and lyophilized protein pharmaceuticals.

Proteins also are subject to a variety of chemical modification and/or degradation reactions such as deamidation, isomerization, hydrolysis, disulfide scrambling, beta-elimination, oxidation and adduct formation. The principal hydrolytic mechanisms of degradation include peptide bond hydrolysis, deamidation of asparagine and glutamine, isomerization of aspartic acid and cyclization of glutamic acid leading to pyro-glutamic acid. A common feature of the hydrolytic degradation pathways is that one significant formulation variable, with respect to the rates of the reactions, is the solution pH.

For example, the hydrolysis of peptide bonds can be acid or base catalyzed. Asparagine and glutamine deamidation also are acid catalyzed below a pH of about 4. Asparagine deamidation at neutral pH occurs through a succinimidyl intermediate that is base catalyzed. The isomerization and racemization of aspartic acid residues can be rapid in slightly acidic to neutral pH (pH 4 - 8). In addition to the generalized pH effects, buffer salts and other excipients can affect the rates of the hydrolytic reactions.

Other exemplary degradation pathways include beta-elimination reactions, which can occur under alkaline pH conditions and lead to racemization or loss of part of the side-chain for certain amino acids. Oxidations of methionine, cysteine, histidine, tyrosine and tryptophan residues are exemplary covalent degradation pathways for proteins.

Because of the number and diversity of different reactions that can result in protein instability the composition of components in a formulation can significantly affect the extent of protein degradation and, consequently, the safety and efficacy of the therapeutic. The formulation of a polypeptide also can affect the ease and frequency of administration and pain upon injection. For example, immunogenic reactions have not only been attributed to protein aggregates but also to mixed aggregates of the therapeutic protein with an inactive component contained in the formulation (Schellekens, H., Nat. Rev. Drug Discov. 1:457-62(2002); Hesmeling, et al., Pharm. Res. 22:1997-2006 (2005)).

However, despite the advances made in the utilization of proteins in therapeutic treatments and the knowledge of the instability process they can undergo, there is still a need to develop formulations with enhanced long-term stability characteristics. A formulation that retains long-term stability under a variety of conditions would provide an effective means of delivering an efficacious and safe amount of the polypeptide. Retention of long-term stability in a formulation also would lower the production and treatment costs. Numerous recombinant or natural proteins could benefit from such consistently stable formulations and thereby provide more effective clinical results.

Thus, there exists a need for formulations that retain long-term stability under a variety of different manufacturing and storage conditions. The present invention satisfies this need and provides related advantages as well.

WO 2004/039826 A1 discloses anti-IL6 antibodies.

US 2004/191243 A1 discloses stabilized antibody formulations.

WO 03/039485 A2 discloses stable liquid pharmaceutical formulations comprising high antibody concentrations.

WO 2006/096461 A2 comprises compositions of anti-M-CSF antibodies.

WO 2006/107854 A2 discloses anti-epidermal growth factor receptor specific binding agents.

WO 2007/145862 A1 discloses HER dimerization inhibitors, such as pertuzumab.

WO 02/096457 A2 discloses stable liquid antibody formulations.

### SUMMARY OF THE INVENTION

The invention provides a formulation comprising an acetic acid buffer, a glutamic acid buffer or a succinic acid buffer with a pH between 4.5 - 5.5, at least one excipient comprising a sugar or a polyol, and an effective amount of a therapeutic antibody having specific binding activity to human epidermal growth factor receptor (EGFR), wherein said therapeutic antibody retains at least about 80% stability for up to two months in solution, and wherein said human antibody is panitumumab. The buffer can include a sodium salt of acetate, glutamate or succinate and the sugar or polyol can include glycerol, sucrose, trehalose or sorbitol.

### BRIEF DESCRIPTION OF THE DRAWINGS

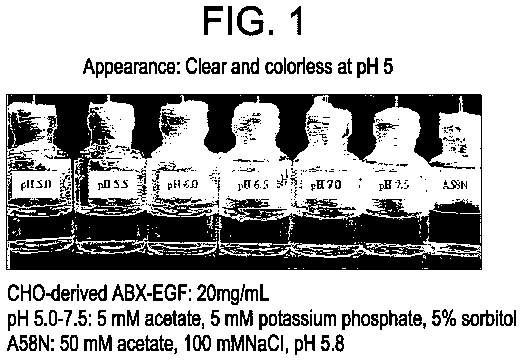

Figure 1 shows the visual appearance of an antibody formulated at various pH's from 5.0 to 7.5.

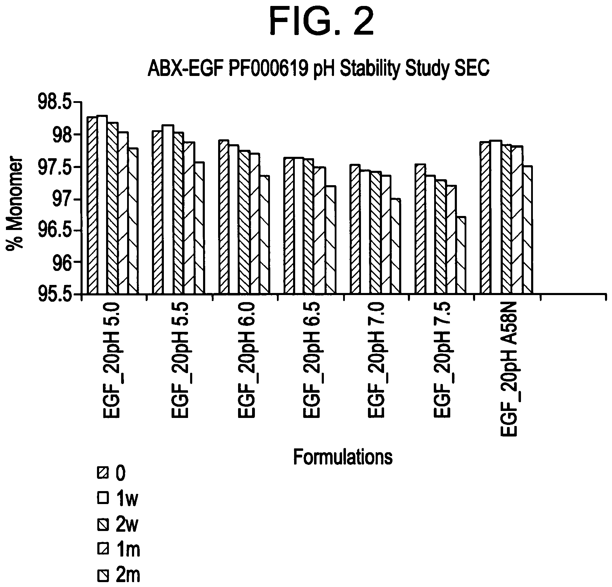

Figure 2 shows SE-HPLC results for the pH stability of an antibody formulation stored at 37C for up to 2 months. Histogram sets for each measured pH correspond from left to right to storage periods of no storage (0); 1 week (1w); 2 weeks (2w); 1 month (1m), and 2 months (2m). For each time point, the pH values corresponded to 5.0, 5.5, 6.0, 6.5, 7.0 and 7.5.

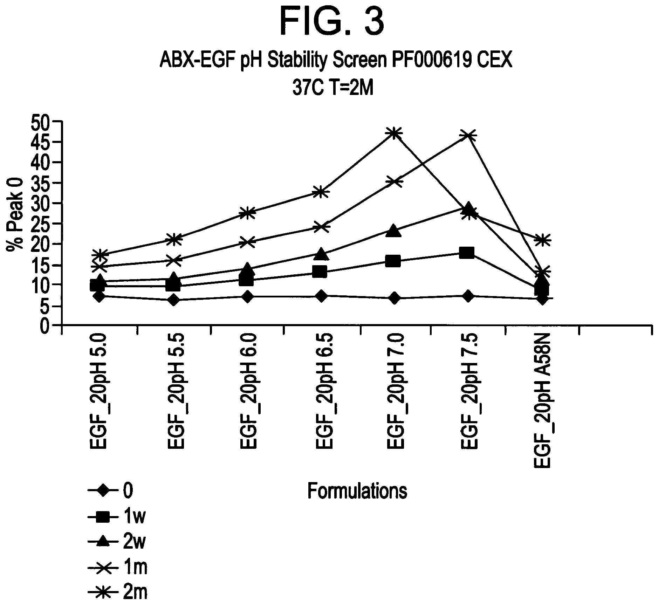

Figure 3 shows the cation exchange chromatography results of an antibody formulated at various pH after storage at 37C for up to 2 months. Storage conditions corresponded to no storage (0, diamonds); 1 week (1w, squares); 2 weeks (2w, triangles); 1 month (1m, X), and 2 months (2m, stars).

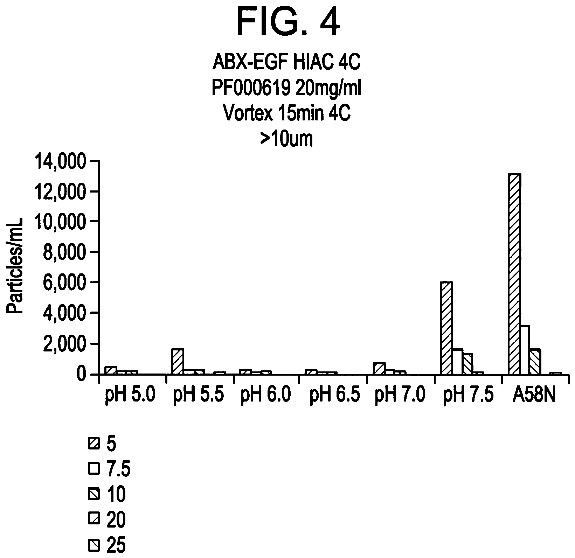

Figure 4 shows the particle counts of an antibody formulated at various pH's after vortexing for 15 minutes at 4C. Histogram sets for each indicated particle size correspond from left to right to 5 µm (5); 7.5 µm (7.5); 10 µm (10); 20 µm (20), and 25 µm (25).

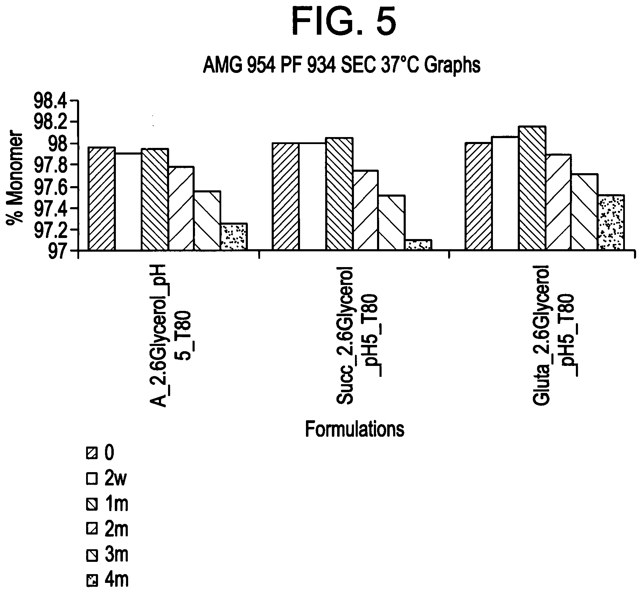

Figure 5 shows size exclusion chromatography results of an antibody formulated in different formulations after storage at 37C for up to 4 months. Histogram sets for each formulation correspond from left to right to storage periods of no storage (0); 2 weeks (2w); 1 month (1m); 2 months (2m); 3 months (3m), and 4 months (4m).

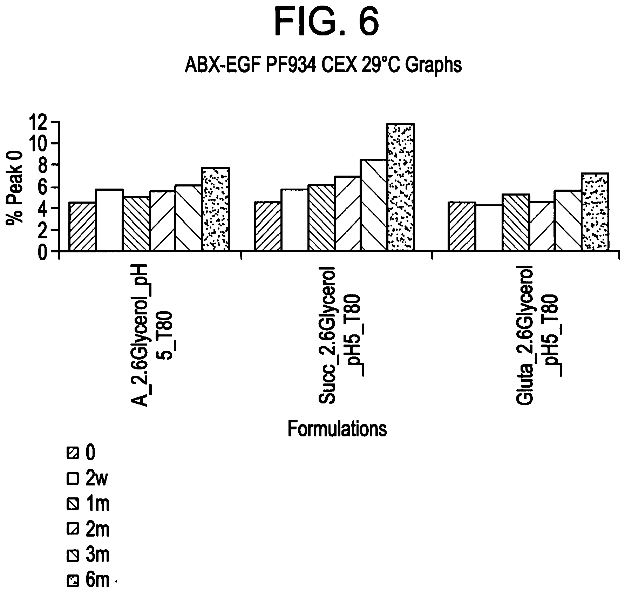

Figure 6 shows the cation exchange chromatography of an antibody formulated in different formulations after storage at 29C for up to 6 months. Histogram sets for each formulation correspond from left to right to storage periods of no storage (0); 2 weeks (2w); 1 month (1m); 2 months (2m); 3 months (3m), and 6 months (6m).

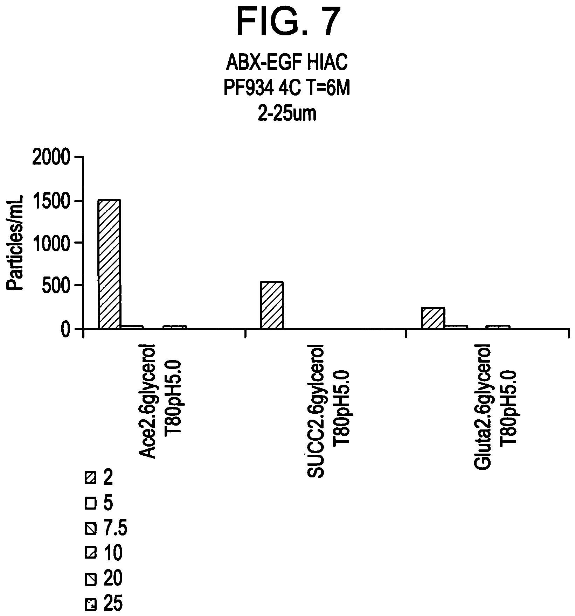

Figure 7 shows the HIAC subvisible particle count of an antibody of different antibody formulations following storage at 4C for 6 months. Histogram sets for each indicated particle size correspond from left to right to 2 µm (2); 5 µm (5); 7.5 µm (7.5); 10 µm (10); 20 µm (20), and 25 µm (25).

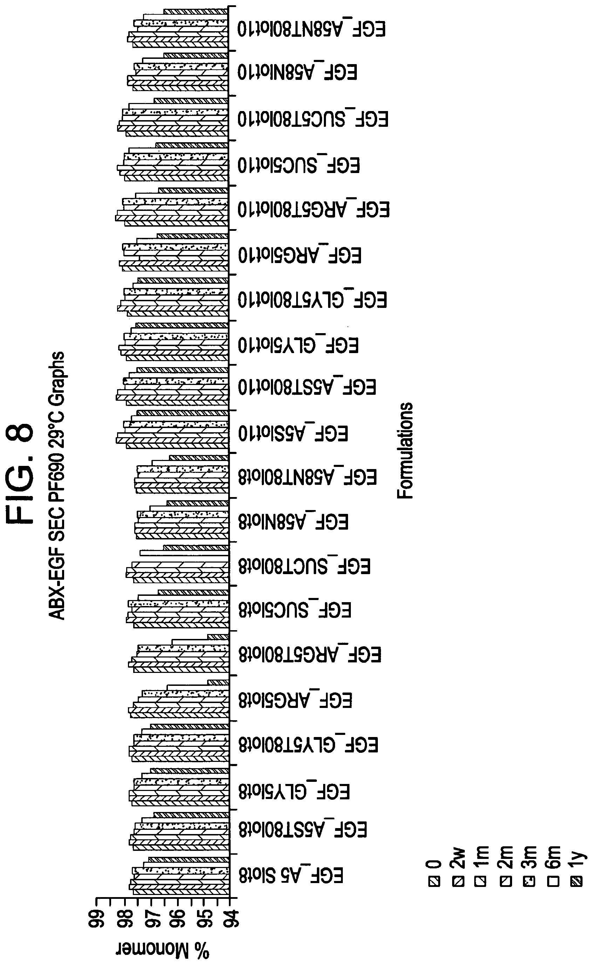

Figure 8 shows size exchange chromatography (SEC)-HPLC measurements of antibody monomer content resulting from various formulations containing different excipients. Histogram sets for each formulation correspond from left to right to storage periods of no storage (0); 2 weeks (2w); 1 month (1m); 2 months (2m); 3 months (3m); 6 months (6m), and 1 year (1y).

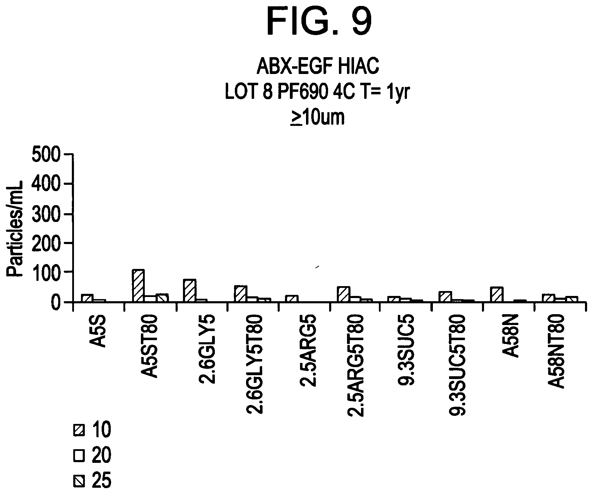

Figure 9 shows HIAC subvisible particle measurements greater than 10 µm of different antibody formulations stored at 4C for 1 year. Histogram sets for each indicated particle size correspond from left to right to 10 µm (10); 20 µm (20), and 25 µm (25).

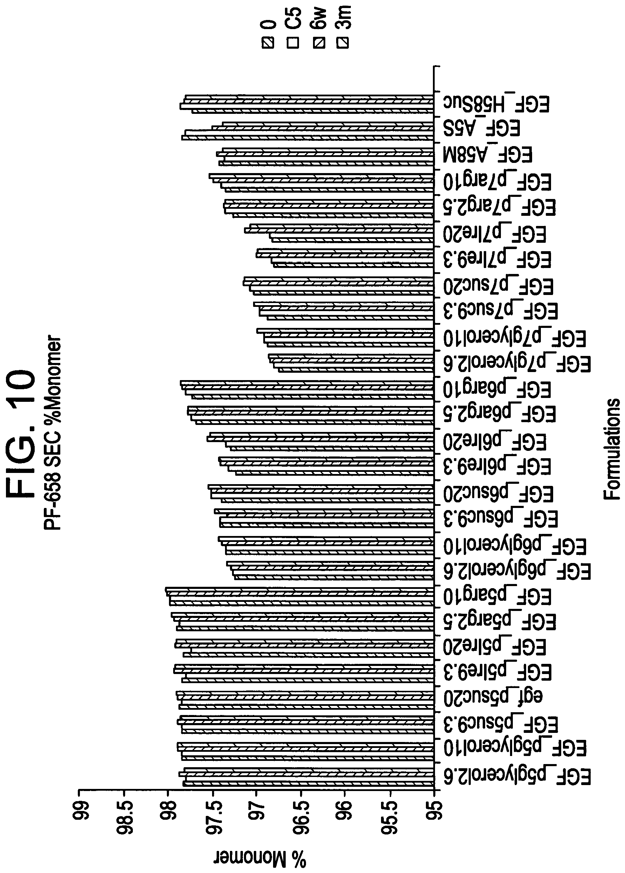

Figure 10 shows the SE-HPLC measurements of antibody monomer content following storage at 30C for up to 3 months in various formulations having a pH ranging from 5.0 to 7.0 and containing different excipients. Histogram sets for each formulation correspond from left to right to storage periods of no storage (0); 5 times freeze and thaw with no months or weeks of -30C storage (C5); 6 weeks (6w), and 3 months (3m).

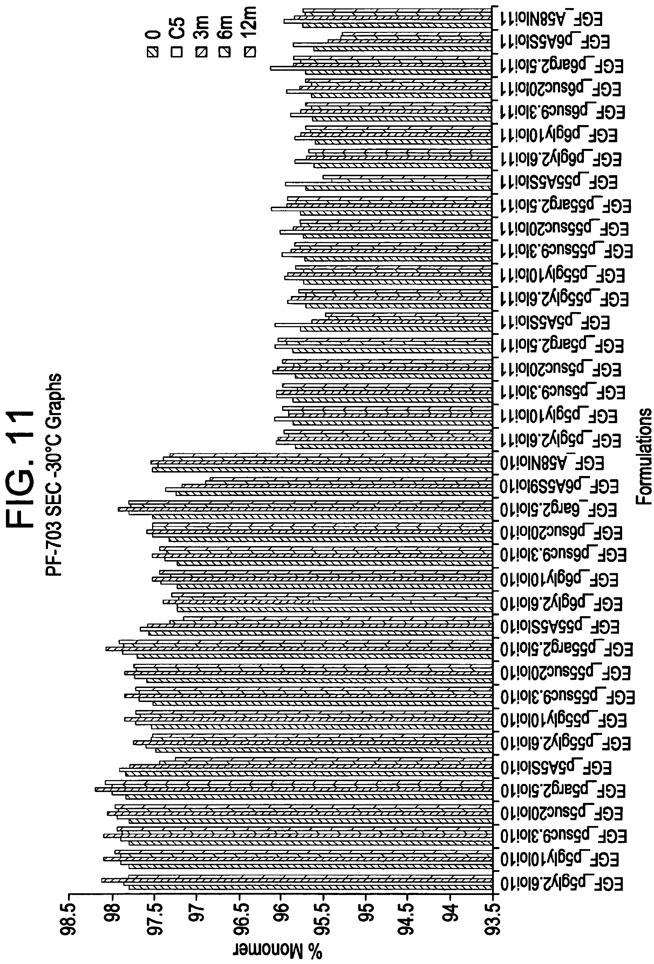

Figure 11 shows the SE-HPLC measurements of antibody monomer content following storage at -30C for up to 1 year in either acetate or phosphate buffer in various formulations having a pH ranging from 5.0 to 6.0 and containing different stabilizers. Histogram sets for each formulation correspond from left to right to storage periods of no storage (0); 5 times freeze and thaw with no months or weeks of -30C storage (C5); 3 months (3m); 6 months (6m), and 12 months (12m).

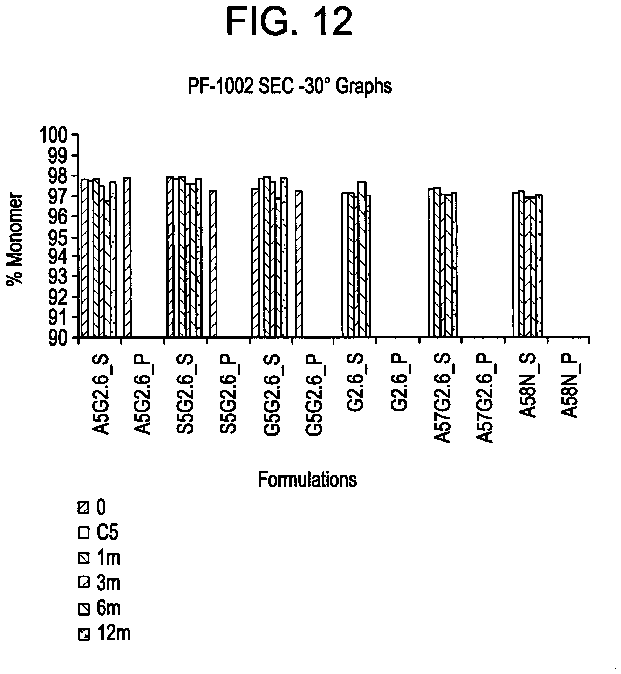

Figure 12 shows the SE-HPLC measurements of antibody monomer content of different formulations following storage at -30C for up to 1 year in either stainless steel or polypropylene containers. Histogram sets for each formulation correspond from left to right to storage periods of no storage (0); 5 times freeze and thaw with no months or weeks of - 30C storage (C5); 1 month (1m); 3 months (3m); 6 months (6m), and 12 months (12m).

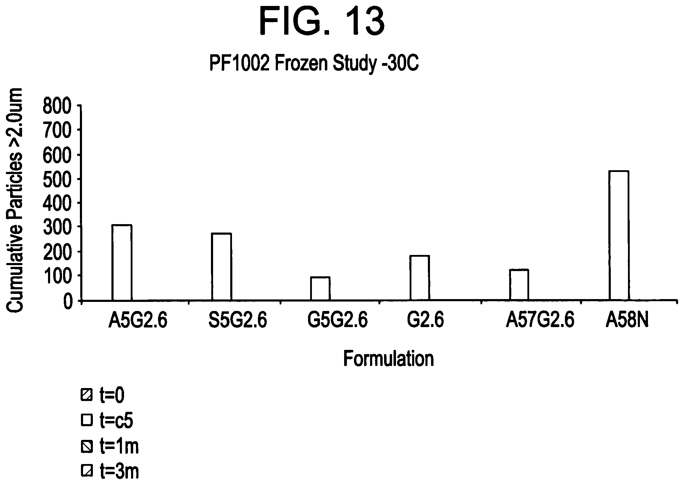

Figure 13 shows the effect of freeze/thawing and storage at -30C on particle formation of various antibody formulations. Histogram sets for each formulation correspond from left to right to storage periods of no storage (t=0); 5 times freeze and thaw with no months or weeks of -30C storage (t=c5); 1 month (t=1m), and 3 months (t=3m).

### DETAILED DESCRIPTION OF THE INVENTION

This invention is directed to a formulation that exhibits optimal stabilizing capacity of the human antibody panitumumab. The formulation contains a acetate, glutamate or succinate buffer system that is particularly useful in pH ranges between 4.5 - 5.5. Panitumumab solubilized or included in a formulation of the invention exhibit stability for long periods of time, allowing administration of a safe and effective amounts of panitumumab. The invention includes patumumab in a formulation having an acetic acid, glutamic acid or succinic acid buffer system. The weak acid component of the buffer system is supplied by a salt form acetate, glutamate or succinate to buffer the formulation and is present at a concentration between about 1-100 mM. The buffering system of the invention maintains polypeptide stability between a pH range of about 4.5-5.5 more particularly between about 4.8-5.3. In this specific embodiment, the formulated patumumab also can contain excipients such as glycerol, sucrose or trehalose. One formulation of the invention is an aqueous solution that exhibits a pH of about 5.0 and maintains buffering capacity in the presence of panitumumab. Another formulation of the invention is a solution that exhibits a pH of about 5.0, maintains buffering capacity in the presence of panitumumab at least about 12-18 months at temperatures below freezing and reduces freeze-thaw-induced aggregation and/or particle formation.

As used herein, the term "biopharmaceutical" is intended to mean a macromolecule or biopolymer such as a polypeptide, nucleic acid, carbohydrate or lipid, or building block thereof, that is intended for use as a pharmaceutical. A "biopharmaceutical formulation" refers to a pharmaceutically acceptable medium that is compatible with a biopharmaceutical and is safe and non-toxic when administered to humans.

As used herein, the term "antibody" is intended to mean a polypeptide product of B cells within the immunoglobulin class of polypeptides which is composed of heavy and light chains and able to bind with a specific molecular target or antigen. The term "monoclonal antibody" refers to an antibody that is the product of a single cell clone or hybridoma. The term also is intended to refer to an antibody produced recombinant methods from heavy and light chain encoding immunoglobulin genes to produce a single molecular immunoglobulin species. Amino acid sequences for antibodies within a monoclonal antibody preparation are substantially homogeneous and the binding activity of antibodies within such a preparation exhibit substantially the same antigen binding activity. As described further below, antibody and monoclonal antibody characteristics are well known in the art.

Monoclonal antibodies can be prepared using a wide variety of methods known in the art including the use of hybridoma, recombinant, phage display and combinatorial antibody library methodologies, or a combination thereof. For example, monoclonal antibodies can be produced using hybridoma techniques including those known in the art and taught, for example, in Harlow and Lane., Antibodies: A Laboratory Manual, Cold Spring Harbor Laboratory Press (1989); Hammerling, et al., in: Monoclonal Antibodies and T-Cell Hybridomas 563-681, Elsevier, N.Y. (1981); Harlow et al., Using Antibodies: A Laboratory Manual, Cold Spring Harbor Laboratory Press (1999), and Antibody Engineering: A Practical Guide, C.A.K. Borrebaeck, Ed., W.H. Freeman and Co., Publishers, New York, pp. 103-120 (1991). Examples of known methods for producing monoclonal antibodies by recombinant, phage display and combinatorial antibody library methods, including libraries derived from immunized and naive animals can be found described in Antibody Engineering: A Practical Guide, C.A.K. Borrebaeck, Ed., supra*.* The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

As used herein, the term "functional fragment" when used in reference to an antibody is intended to mean a portion of an antibody which still retains some or all of its specific antigen binding activity. Such functional fragments can include, for example, antibody functional fragments such as Fd, Fv, Fab, F(ab'), F(ab)₂, F(ab')₂, single chain Fv (scFv), chimeric antibodies, diabodies, triabodies, tetrabodies and minibody. Other functional fragments can include, for example, heavy (H) or light (L) chain polypeptides, variable heavy (V_{H}) and variable light (V_{L}) chain region polypeptides, complementarity determining region (CDR) polypeptides, single domain antibodies, and polypeptides that contain at least a portion of an immunoglobulin that is sufficient to retain its specific binding activity. Such antibody binding fragments can be found described in, for example, Harlow and Lane, *supra;* Molec. Biology and Biotechnology: A Comprehensive Desk Reference (Myers, R.A. (ed.), New York: VCH Publisher, Inc.); Huston et al., Cell Biophysics, 22:189-224 (1993); Plückthun and Skerra, Meth. Enzymol., 178:497-515 (1989) and in Day, E.D., Advanced Immunochemistry, Second Ed., Wiley-Liss, Inc., New York, NY (1990).

With respect to antibodies and functional fragments thereof, various forms, alterations and modifications are well known in the art. The monoclonal antibodies of the invention can include any of such various monoclonal antibody forms, alterations and modifications. Examples of such various forms and terms as they are known in the art are set forth below.

A Fab fragment refers to a monovalent fragment consisting of the V_{L}, V_{H}, C_{L} and C_{H}1 domains; a F(ab')₂ fragment is a bivalent fragment comprising two Fab fragments linked by a disulfide bridge at the hinge region; a Fd fragment consists of the V_{H} and C_{H}1 domains; an Fv fragment consists of the V_{L} and V_{H} domains of a single arm of an antibody; and a dAb fragment (Ward et al., Nature 341:544-546, (1989)) consists of a V_{H} domain.

An antibody can have one or more binding sites. If there is more than one binding site, the binding sites may be identical to one another or may be different. For example, a naturally occurring immunoglobulin has two identical binding sites, a single-chain antibody or Fab fragment has one binding site, while a "bispecific" or "bifunctional" antibody has two different binding sites.

A single-chain antibody (scFv) refers to an antibody in which a V_{L} and a V_{H} region are joined via a linker (*e.g.,* a synthetic sequence of amino acid residues) to form a continuous polypeptide chain wherein the linker is long enough to allow the protein chain to fold back on itself and form a monovalent antigen binding site (see, *e.g.,* Bird et al., Science 242:423-26 (1988) and Huston et al., Proc. Natl. Acad. Sci. USA 85:5879-83 (1988)). Diabodies refer to bivalent antibodies comprising two polypeptide chains, wherein each polypeptide chain comprises V_{H} and V_{L} domains joined by a linker that is too short to allow for pairing between two domains on the same chain, thus allowing each domain to pair with a complementary domain on another polypeptide chain (see, *e*.*g*., Holliger et al., Proc. Natl. Acad. Sci. USA 90:6444-48 (1993), and Poljak et al., Structure 2:1121-23 (1994)). If the two polypeptide chains of a diabody are identical, then a diabody resulting from their pairing will have two identical antigen binding sites. Polypeptide chains having different sequences can be used to make a diabody with two different antigen binding sites. Similarly, tribodies and tetrabodies are antibodies comprising three and four polypeptide chains, respectively, and forming three and four antigen binding sites, respectively, which can be the same or different.

A CDR refers to a region containing one of three hypervariable loops (H1, H2 or H3) within the non-framework region of the immunoglobulin (Ig or antibody) V_{H} β-sheet framework, or a region containing one of three hypervariable loops (L1, L2 or L3) within the non-framework region of the antibody V_{L} β-sheet framework. Accordingly, CDRs are variable region sequences interspersed within the framework region sequences. CDR regions are well known to those skilled in the art and have been defined by, for example, Kabat as the regions of most hypervariability within the antibody variable (V) domains (Kabat et al., J. Biol. Chem. 252:6609-6616 (1977); Kabat, Adv. Prot. Chem. 32:1-75 (1978)). CDR region sequences also have been defined structurally by Chothia as those residues that are not part of the conserved β-sheet framework, and thus are able to adapt different conformations (Chothia and Lesk, J. Mol. Biol. 196:901-917 (1987)). Both terminologies are well recognized in the art. The positions of CDRs within a canonical antibody variable domain have been determined by comparison of numerous structures (Al-Lazikani et al., J. Mol. Biol. 273:927-948 (1997); Morea et al., Methods 20:267-279 (2000)). Because the number of residues within a loop varies in different antibodies, additional loop residues relative to the canonical positions are conventionally numbered with a, b, c and so forth next to the residue number in the canonical variable domain numbering scheme (Al-Lazikani et al., *supra* (1997)). Such nomenclature is similarly well known to those skilled in the art.

For example, CDRs defined according to either the Kabat (hypervariable) or Chothia (structural) designations, are set forth in the table below.

**Table: CDR Definitions**

| | Kabat¹ | Chothia² | Loop Location |
|---|---|---|---|
| V_{H} CDR1 | 31-35 | 26-32 | linking Band C strands |
| V_{H} CDR2 | 50-65 | 53-55 | linking C' and C" strands |
| V_{H} CDR3 | 95-102 | 96-101 | linking F and G strands |
| V_{L} CDR1 | 24-34 | 26-32 | linking B and C strands |
| V_{L} CDR2 | 50-56 | 50-52 | linking C' and C" strands |
| V_{L} CDR3 | 89-97 | 91-96 | linking F and G strands |

| | | | |
|---|---|---|---|
| ¹ Residue numbering follows the nomenclature of Kabat et al., *supra* ² Residue numbering follows the nomenclature of Chothia et al., *supra* | | | |

A chimeric antibody refers to an antibody that contains one or more regions from one antibody and one or more regions from one or more other antibodies. In one specific example, one or more of the CDRs are derived from a non-human donor antibody having specific activity to EGFR and the variable region framework is derived from a human recipient antibody. In another specific example, all of the CDRs are derived from a non-human donor antibody having specific activity to EGFR and the variable region framework is derived from a human recipient antibody. In yet another specific example, the CDRs from more than one non-human EGFR-specific antibodies are mixed and matched in a chimeric antibody. For instance, a chimeric antibody can include a CDR1 from the light chain of a first non-human EGFR-specific antibody, a CDR2 and a CDR3 from the light chain of a second non-human EGFR-specific antibody and the CDRs from the heavy chain from a third EGFR-specific antibody. Further, the framework regions can be derived from one of the same or from one or more different human antibodies or from a humanized antibody. Chimeric antibodies can be produced where both the donor and recipient antibodies are human.

A humanized antibody or grafted antibody has a sequence that differs from a non-human species antibody sequence by one or more amino acid substitutions, deletions, and/or additions, such that the humanized antibody is less likely to induce an immune response, and/or induces a less severe immune response, as compared to the non-human species antibody, when it is administered to a human subject. In one specific example, certain amino acids in the framework and constant domains of the heavy and/or light chains of the non-human species antibody are changed to produce the humanized antibody. In another specific example, the constant domain(s) from a human antibody are fused to the variable domain(s) of a non-human species. Examples of how to make humanized antibodies may be found in U.S. Pat. Nos. 6,054,297, 5,886,152 and 5,877,293. Humanized antibodies also include antibodies produced using antibody resurfacing methods and the like.

A human antibody refers to antibodies that have one or more variable and constant regions derived from human immunoglobulin sequences. For example, a fully human antibody includes an antibody where all of the variable and constant domains are derived from human immunoglobulin sequences. Human antibodies can be prepared using a variety of methods known in the art. A specific example of a human antibody is panitumumab, which is the subject matter of the human anti-EGFR antibody described in U.S. Patent No. 6,235,883. Panitumumab also is known in the art as Vcctibix^{Tm} (Amgen, Thousand Oaks, California) and is useful for treating pathological conditions such as metastatic colorectal cancer, for example.

One or more CDRs also can be incorporated into a molecule either covalently or noncovalently to make it an immunoadhesin. An immunoadhesin can incorporate the CDR(s) as part of a larger polypeptide chain, can covalently link the CDR(s) to another polypeptide chain, or can incorporate the CDR(s) noncovalently. The CDRs permit the immunoadhesin to specifically bind to a particular antigen of interest.

A neutralizing antibody or an inhibitory antibody when used in reference to an formulated antibody of the invention refers to an antibody that inhibits the binding of receptor to ligand. In the specific example of an EGFR-speicific monoclonal antibody, an inhibitory antibody refers to a monoclonal antibody that inhibits the binding of EGFR to EGF when an excess of the EGFR-specific antibody reduces the amount of EGF bound to EGFR. Binding inhibition can occur by at least 10%, particularly by at least about 20%. In various specific examples, the monoclonal antibody can reduce the amount of EGF bound to EGFR by, for example, at least 30%, 40%, 50%, 60%, 70%, 75%, 80%, 85%, 90%, 95%, 97%, 99%, and 99.9%. The binding reduction may be measured by any means known to one of ordinary skill in the art, for example, as measured in an *in vitro* competitive binding assay.

An "antagonistic" antibody refers to an antibody that inhibits an activity response of its antigen. In the specific example of an EGFR-specific monoclonal antibody, an antagonistic antibody refers to an antibody that inhibits the activity of EGFR when added to a cell, tissue or organism expressing EGFR. Diminution in activity can be by at least about 5%, particularly by at least about 10%, more particularly, by at least about 15% or more, compared to the level of EGFR activity in the presence of EGF alone. In various specific examples, the EGFR-specific monoclonal antibodies of the invention can inhibit the EGFR activity by at least about 20%, 30%, 40%, 50%, 60%, 70%, 80%, 90% or 100%.

An agonist antibody refers to an antibody that activates an activity response of its antigen. In the specific example of an EGFR-specific monoclonal antibody, an agonist antibody refers to an antibody that activates EGFR by at least about 5%, particularly by at least about 10%, more particularly, by at least about 15% when added to a cell, tissue or organism expressing EGFR, where "100% activation" is the level of activation achieved under physiological conditions by the same molar amount of EGF. In various specific examples, the KGFR-specific monoclonal antibodies of the invention can activate EGFR activity by at least about 20%, 30%, 40%, 50%, 60%, 70%, 80%, 90%, 100%, 125%, 150%, 175%, 200%, 250%, 300%, 350%, 400%, 450%, 500%, 750% or 1000%.

An epitope refers to a part of a molecule, for example, a portion of a polypeptide, that specifically binds to one or more antibodies within the antigen binding site of the antibody. Epitopic determinants can include continuous or non-continuous regions of the molecule that binds to an antibody. Epitopic determinants also can include chemically active surface groupings of molecules such as amino acids or sugar side chains and have specific three dimensional structural characteristics and/or specific charge characteristics.

As used herein, the term "specific" when used in reference to a monoclonal antibody binding activity is intended to mean that the referenced monoclonal antibody exhibits preferential binding for its antigen compared to other similar antigens. In the specific example of an EGFR-specific monoclonal antibody, specific binding activity is intended to mean that the referenced EGFR monoclonal antibody exhibits preferential binding for EGFR compared to other receptors related to epidermal growth factor. Preferential binding includes a monoclonal antibody of the invention exhibiting detectable binding to EGFR while exhibiting little or no detectable binding to another a related growth factor receptor.

As used herein, the term "epidermal growth factor receptor" or "EGFR" is intended to mean the art receptor that can be found expressed on the surface of epidermal cells and with binds to epidermal growth factor (EGF) and/or transforming growth factor alpha (TGFα). This receptor is well known in the art and can be found described in, for example, Yarden, Y., and Sliwkowski, M. X., Nat Rev Mol Cell Biol. 2,127-37 (2001), and Mendelsohn, J. and Baselga, J., J Clin Oncol 21, 2787-99 (2003). EGFR also is the antigen for the panitumumab human antibody, which is the subject matter of U.S. Patent No. 6,235,883.

As used herein, the term "acetic acid" is intended to mean a acid having the formula CH₃COOH. Acetic acid has a melting point of 16.7°C and boiling point of 118.0°C.

As used herein, the term "glutamic acid" is intended to mean an acidic amino acid having the formula C₅H₉NO₄. The term includes both L and D forms of the amino acid As used herein, the term "succinic acid" is intended to mean a dicarboxylic acid having the formula C₄H₆O₄. Succinic acid has a melting point of melting point of 185 °C and a boiling point of 235 °C.

An "acetic acid" or "acetate buffer," a "glutamic acid" or "glutamate buffer" or an "succinic acid" or "succinate buffer" as the terms are used herein are intended to refer to a buffer containing acetic acid, glutamic acid or succinic acid in equilibrium with its respective conjugate base. Each of these buffers can provide optimal buffer capacity in the region of their pKₐ, where buffer capacity refers to a resistance to change in pH when perturbed with either acid or base added to the solution. The pKₐ of acetic acid is 4.75. The pKₐ the glutamic acid sidechain is 4.07 whereas the pKₐ of succinic acid is 4.19 and 5.57 for its two carboxylic acid moieties. The acetic acid form of an acetic acid buffer of the invention can include, for example, acetic acid, acetate ion and/or acetate including acetic acid salt forms. Similarly, the glutamic acid form of aglutamic acid buffer of the invention can include, for example, glutamic acid, glutamate ion and/or glutamate including glutamic acid salt forms. Further, the succinic acid form of a succinic acid buffer of the invention can include, for example, succinic acid, succinate ion and/or succinate including succinic acid salt forms. Exemplary salts that can be included in the buffer of the invention include, for example, sodium, potassium, calcium, organic amino or magnesium salt. Acetic acid, acetic acid buffers, glutamic acid, glutamic acid buffers, succinic acid and succinic acid buffers are well known by those skilled in the art.

As used herein, the term "excipient" is intended to mean a therapeutically inactive substance. Excipients can be included in a formulation for a wide variety of purposes including, for example, as a diluent, vehicle, buffer, stabilizer, tonicity agent, bulking agent, surfactant, cryoprotectant, lyoprotectant, anti-oxidant, metal ion source, chelating agent and/or preservative. Excipients include, for example, polyols such as sorbitol or mannitol; sugars such as sucrose, lactose or dextrose; polymers such as polyethylene glycol; salts such as NaCl, KCl or calcium phosphate, amino acids such as glycine, methionine or glutamic acid, surfactants, metal ions, buffer salts such as propionate, acetate or succinate, preservatives and polypeptides such as human serum albumin, as well as saline and water. Particularly useful excipients of the invention include sugars including sugar alcohols, reducing sugars, non-reducing sugars and sugar acids. Excipients are well known in the art and can be found described in, for example, Wang W., Int. J. Pharm. 185:129-88 (1999) and Wang W., Int. J. Pharm. 203:1-60 (2000).

Briefly, sugar alcohols, also known as a polyols, polyhydric alcohols, or polyalcohols, are hydrogenated forms of carbohydrate having a carbonyl group reduced to a primary or secondary hydroxyl group. Polyols can be used as stabilizing excipients and/or isotonicity agents in both liquid and lyophilized formulations. Polyols can protect polypeptides from both physical and chemical degradation pathways. Preferentially excluded co-solvents increase the effective surface tension of solvent at the protein interface whereby the most energetically favorable structural conformations are those with the smallest surface areas. Specific examples of sugar alcohols include sorbitol, glycerol, mannitol, xylitol, maltitol, lactitol, erythritol and threitol.

Reducing sugars include, for example, sugars with a ketone or aldehyde group and contain a reactive hemiacetal group, which allows the sugar to act as a reducing agent. Specific examples of reducing sugars include fructose, glucose, glyceraldehyde, lactose, arabinose, mannose, xylose, ribose, rhamnose, galactose and maltose.

Non-reducing sugars contain an anomeric carbon that is an acetal and is not substantially reactive with amino acids or polypeptides to initiate a Maillard reaction. Sugars that reduce Fehling's solution or Tollen's reagent also are known as reducing sugars. Specific examples of non-reducing sugars include sucrose, trehalose, sorbose, sucralose, melezitose and raffinose.

Sugar acids include, for example, saccharic acids, gluconate and other polyhydroxy sugars and salts thereof.

Buffer excipients maintain the pH of liquid formulations through product shelf-life and maintain the pH of lyophilized formulations during the lyophilization process and upon reconstitution, for example.

Tonicity agents and/or stabilizers included in liquid formulations can be used, for example, to provide isotonicity, hypotonicity or hypertonicity to a formulation such that it is suitable for administration. Such excipients also can be used, for example, to facilitate maintenance of a polypeptides' structure and/or to minimize electrostatic, solution protein-protein interactions. Specific examples of tonicity agents and/or stabilizers include polyols, salts and/or amino acids. Tonicity agents and/or stabilizers included in lyophilized formulations can be used, for example, as a cryoprotectant to guard polypeptides from freezing stresses or as a lyoprotectant to stabilize polypeptides in the freeze-dried state. Specific examples of such cryo- and lyoprotectants include polyols, sugars and polymers.

Bulking agents are useful in lyophilized formulations to, for example, enhance product elegance and to prevent blowout. Bulking agents provide structural strength to the lyo cake and include, for example, mannitol and glycine.

Anti-oxidants are useful in liquid formulations to control protein oxidation and also can be used in lyophilized formulations to retard oxidation reactions.

Metal ions can be included in a liquid formulation, for example, as a co-factor and divalent cations such as zinc and magnesium can be utilized in suspension formulations. Chelating agents included in liquid formulations can be used, for example, to inhibit metal ion catalyzed reactions. With respect to lyophilized formulations, metal ions also can be included, for example, as a co-factor. Although chelating agents are generally omitted from lyophilized formulations, they also can be included as desired to reduce catalytic reactions during the lyophilization process and upon reconstitution.

Preservatives included in liquid and/or lyophilized formulations can be used, for example, to protect against microbial growth and are particularly beneficial in multi-dose formulations. In lyophilized formulations, preservatives are generally included in the reconstitution diluent. Benzyl alcohol is a specific example of a preservative useful in a formulation of the invention.

As used herein, the term "surfactant" is intended to mean a substance that functions to reduce the surface tension of a liquid in which it is dissolved. Surfactants can be included in a formulation for a variety of purposes including, for example, to prevent or control aggregation, particle formation and/or surface adsorption in liquid formulations or to prevent or control these phenomena during the lyophilization and/or reconstitution process in lyophilized formulations. Surfactants include, for example, amphipathic organic compounds that exhibit partial solubility in both organic solvents and aqueous solutions. General characteristics of surfactants include their ability to reduce the surface tension of water, reduce the interfacial tension between oil and water and also form micelles. Surfactants of the invention include non-ionic and ionic surfactants. Surfactants are well known in the art and can be found described in, for example, Randolph T.W. and Jones L.S., Surfactant-protein interactions. Pharm Biotechnol. 13:159-75 (2002).

Briefly, non-ionic surfactants include, for example, alkyl poly (ethylene oxide), alkyl polyglucosides such as octyl glucoside and decyl maltoside, fatty alcohols such as cetyl alcohol and oleyl alcohol, cocamide MEA, cocamide DEA, and cocamide TEA. Specific examples of non-ionic surfactants include the polysorbates including, for example, polysorbate 20, polysorbate 28, polysorbate 40, polysorbate 60, polysorbate 65, polysorbate 80, polysorbate 81, polysorbate 85 and the like; the poloxamers including, for example, poloxamer 188, also known as poloxalkol or poly(ethylene oxide)-poly(propylene oxide), poloxamer 407 or polyethylene-polypropylene glycol and the like, and polyethylene glycol (PEG). Polysorbate 20 is synonymous with TWEEN 20, sorbitan monolaurate and polyoxyethylenesorbitan monolaurate.

Ionic surfactants include, for example, anionic, cationic and zwitterionic surfactants. Anionic surfactants include, for example, sulfonate-based or carboxylate-based surfactants such as soaps, fatty acid salts, sodium dodecyl sulfate (SDS), ammonium lauryl sulfate and other alkyl sulfate salts. Cationic surfactants include, for example, quaternary ammonium-based surfactants such as cetyl trimethylammonium bromide (CTAB), other alkyltrimethylammonium salts, cetyl pyridinium chloride, polyethoxylated tallow amine (POEA) and benzalkonium chloride. Zwitterionic or amphoteric surfactants include, for example, dodecyl betaine, dodecyl dimethylamine oxide, cocamidopropyl betaine and coco ampho glycinate.

As used herein, the term "therapeutic" when used in reference to a polypeptide as disclosed herein including panitumumab of the invention, is intended to mean that the polypeptide is intended for use in the cure, mitigation, treatment or prevention of disease in a human or other animal. Accordingly, a therapeutic polypeptide is a specific type of pharmaceutical and can include a single polypeptide or two or more polypeptide subunits. A therapeutic polypeptide includes an antibody, a functional antibody fragment thereof, a peptibody or functional fragment thereof, growth factors, cytokines, cell signaling molecules and hormones. A wide variety of therapeutic polypeptides are well know in the art, all of which are included within the meaning of the term as it is used herein. Exemplary therapeutic polypeptides that can be used in a formulation of the invention include, for example, antibodies such as panitumumab of the invention (Vectibix^{Tm}) and Epraluzumab® (Emab) as well as functional fragments to a wide variety of antigens, interleukins, G-CSF, GM-CSF, kinases, TNF and TNFR ligands, cyclins and erythropoietin.

As used herein, the term "effective amount" when used in reference to a therapeutic macromolecule such as a therapeutic polypeptide is intended to mean an amount of the therapeutic molecule sufficient to ameliorate at least one symptom associated with a targeted disease or physiological condition.

A formulation of the invention exhibits optimal properties for administration, storage and manipulation of panitumumab. Manipulation includes, for example, lyophilization, reconstitution, dilution, titration and the like. The buffering component of a formulation of the invention is efficient to prepare and can easily be combined with panitumumab using any of a variety of methods well know in the art, avoiding cumbersome and, sometimes lengthy, preparatory and/or intermediate steps. Additionally, the aqueous acetic acid, glutamic acid or succinic acid buffering components are compatible with a wide variety of excipients and surfactants that facilitate stability of a panitumumab. These and other attributes of a formulation of the invention described herein allow stable formulations of panitumumab to be prepared and maintained over periods exceeding 12-18 months or more.

Stability of a formulation of the invention, including a liquid formulation of the invention, refers to the retention of structure and/or function of panitumumab within a formulation. panitumumab in a formulation of the invention will exhibit attributes such as resistance to change or deterioration that affect stability or function and therefore maintain consistent functional characteristics over time. Accordingly, formulations of the invention will exhibit, for example, reliability and safety with respect to activity per volume or activity units.

In one embodiment, the stability of a panitumumab within a formulation of the invention includes, for example, the retention of physical and/or chemical stability. Polypeptide stability can be assessed by, for example, determining whether the polypeptide has been subjected to a physical degradation and/or chemical degradation pathway such as those described previously, including chemical modification of its structure. Retention in stability of a polypeptide in a formulation of the invention includes, for example, retention of physical or chemical stability between about 80-100%, 85-99%, 90-98%, 92-96% or 94-95% compared to the stability of the polypeptide at an initial time point Accordingly, stability of panitumumab within a formulation of the invention includes retention of stability greater than 99.5%, at least about 99%, 98%, 97%, 96%, 95%, 94%, 93%, 92%, 91%, 90%, 89%, 88%, 87%, 86%, 85%, 84%, 83%, 82%, 81% or 80% compared to the stability of panitumumab at an initial time point.

In a further embodiment, stability of panitumumab within a formulation of the invention includes, for example, retention of activity. Polypeptide activity can be assessed using, for example, an *in vitro,* in *vivo* and/or *in situ* assay indicative of the polypeptide's function. Retention of stability of panitumumab in a formulation of the invention includes, for example, retention of activity between about 50-100% or more, depending on the variability of the assay. For example, retention in stability can include retention of activity between about 60-90% or 70-80% compared to the activity panitumumab at an initial time point Accordingly, stability of panitumumab within a formulation of the invention includes retention of activity of at least about 50%, 55%, 60%, 65%, 70%, 75%, 80%, 85%, 90%, 95% or 100% and can include activity measurements greater than 100% such as 105%, 110%, 115%, 120%, 125% or 150% or more compared to the activity of panitumumab at an initial time point. Generally, an initial time point is selected to be the time that panitumumab is first prepared in a formulation of the invention or first examined for quality (i.e., meets release specifications). An initial time point also can include the time at which panitumumab reformulated in a formulation of the invention. The reformulation can be, for example, at a higher concentration, lower concentration or at the same concentration of an initial preparation.

A formulation of the invention can be prepared to be isotonic with a reference solution or fluid (i.e., blood serum). An isotonic solution has a substantially similar amount of dissolved solute in it compared to the things around it so that it is osmotically stable. Unless expressly compared to a specific solution or fluid, isotonic or isotonicity is exemplary used herein by reference to human blood serum (e.g., 300 mOsmol/kg). Therefore, an isotonic formulation of the invention will contain a substantially similar concentration of solutes or exhibit substantially similar osmotic pressure as human blood. In general, an isotonic solution contains about the same concentration of solutes as normal saline for humans and many other mammals, which is about 0.9 weight percent (0.009 g/ml) salt in aqueous solution (e.g., 0.009 g/ml NaCl). Formulations of the invention also can include hypotonic or hypertonic solution preparations.

A formulation can be prepared in any of a variety of ways well known in the art to produce an acetic acid, glutamic acid or succinic acid buffer component having a desired pH, at least one excipient and an effective amount of panitumumab. In this regard, the buffering capacity of a formulation of the invention is supplied by the weak acids acetic acid, glutamic acid or succinic acid, which exhibit strong buffering capacity at a pH range that is within about 1 pH unit of their respective pKₐs. Acetic acid, glutamic acid or succinic acid have pKₐs which are optimal for many biological molecules including, for example, antibodies such as panitumumab.

The acetic acid, glutamic acid or succinic acid component can be supplied to the buffering system in a variety of different acetic acid, glutamic acid or succinic acid forms. For example, the acetic acid, glutamic acid or succinic acid component can be supplied as their acid, acid salt or any other form that is available or that can be produced using chemical synthesis. The acid salt forms of these acids - acetate, glutamate or succinate - are particularly useful for producing a buffering system of a formulation because they are commercially available in highly purified form. Acetate, glutamate and succinate salts include, for example, those described previously as well as others known in the art. A highly purified form of a formulation component refers to pharmaceutical grade purity level, which is sufficiently pure to administer to a human such that it is devoid of contaminants so as to be safe and non-toxic.

Acetic acid, glutamic acid and succinic acid buffers are well known in by those skilled in the art. A formulation of the invention will contain a concentration of, for example, an acid or acid salt of the invention having sufficient buffering capacity to maintain a selected pH of a formulation at a selected temperature. Useful concentrations of acid or salt (ie, acetic acid or acetate, glutamic acid or glutamate or succinic acid or succinate) include, for example, between about 1-150 mM and as high as 200 mM or more. For example, in some instances, it can be desirable to include up to 1 M acid or acid salt to produce a hypertonic formulation of the invention. Such hypertonic solutions can be diluted to produce an isotonic formulation prior to use if desired. By way of exemplification, useful concentrations of acid or acid salt buffer of the invention include, for example, between about 1-200 mM, 5-175 mM, 10-150 mM, 15-125 mM, 20-100 mM, 25-80 mM, 30-75 mM, 35-70 mM, 40-65 mM and 45-60 mM. Other useful concentrations of acid or acid salt include, for example, between about 1-50 mM, 2-30 mM, 3-20 mM, 4-10 mM and 5-8 mM. Accordingly, an acid or acid salt concentration of about 1, 2, 3, 4, 5, 6, 7, 8, 9, 10, 11, 12, 13, 14, 15, 16, 17, 18, 19 or 20 mM or more also are useful. All values above and below these exemplary concentrations also can be used in a formulation. Therefore, a formulation of the invention can have a acid or acid salt less than 1 mM or greater than 20 mM including, for example, 21, 22, 23, 24, 25, 30, 35, 40,45 or 50 mM or more acid oracid salt. Various formulation are exemplified in the Example below and shown in Figures 1-13.

As described previously, the pKₐ of an acetic acid, glutamic acid or succinate acid buffer in a formulation of the invention is particularly suitable for use with polypeptides because they have strong buffering capacity between about pH 4.5-7, which can be optimal for maintenance of polypeptide stability. A buffer component of a formulation of the invention can be prepared to exhibit any effective buffering capacity within a pH range of between about 4.5 to 7.0. Exemplary pH ranges of an acetic acid, glutamic acid or succinic acid buffer and/or the final formulation can include pH ranges between about 3.5-6.5, between about 4.0-6.0, between about 4.5-5.5, between about 4.8-5.2 or about 5.0. Accordingly, a buffer and/or the final formulation can be prepared to have a pH of about 3.0 or less, about 3.5, 4.0, 4.5, 4.8, 5.0, 5.2, 5.5, 6.0, 6.5 or about 7.0 or more. All pH values above, below and in between these exemplary values also can be used in an acetic acid, glutamic acid or succinic acid buffer and/or the final formulation. Therefore, for example, a buffer component and/or the final formulation of the invention can be prepared to have a pH between 4.5-5.5 and all values within these ranges.

An acetic acid, glutamic acid or succinic acid buffer component of a formulation of the invention can include one or more excipients. As described previously, one role of an included excipient is to provide stabilization of polypeptides against stresses that can occur during manufacturing, shipping and storage. To accomplish this role, at least one excipient can function as a buffer, stabilizer, tonicity agent, bulking agent, surfactant, cryoprotectant, lyoprotectant, anti-oxidant, metal ion source, chelating agent and/or preservative. In addition, at least one excipient also can function as a diluent and/or vehicle or be employed to reduce viscosity in high concentration formulations in order to enable their delivery and/or enhance patient convenience.

Similarly, at least one excipient additionally can confer more than one of the above functions onto a formulation of the invention. Alternatively, two or more excipients can be included in a formulation of the invention to perform more than one of the above or other functions. For example, an excipient can be included as a component in a formulation of the invention to change, adjust or optimize the osmolality of the formulation, thereby acting as a tonicifier. Similarly, a tonicity agent and a surfactant can both be included in a formulation of the invention to both adjust the osmolality and control aggregation. Excipients, their use, formulation and characteristics are well known in the art and can be found described in, for example, Wang W., Int. J. Pharm. 185:129-88 (1999) and Wang W., Int. J. Pharm. 203:1-60 (2000).

In general, excipients can be chosen on the basis of the mechanisms by which they stabilize proteins against various chemical and physical stresses. As described herein, certain excipients are beneficial to include so as to alleviate the effects of a specific stress or to regulate a particular susceptibility of a specific polypeptide. Other excipients are beneficial to include because they have more general effects on the physical and covalent stabilities of proteins. Particularly useful excipients include those chemically and functionally innocuous or compatible with aqueous buffer solutions and polypeptides so as to optimize the stability properties of a formulation. Various such excipients are described herein as exemplary excipients exhibiting chemical compatibility with the aqueous formulations of the invention and functional compatibility with the polypeptide included in such formulations. Those skilled in the art will understand that the teachings and guidance provided herein with respect to the exemplified excipients are equally applicable to the use of a wide range of other excipients well known in the art.

For example, optimal excipients chosen to enhance or confer stability of a polypeptide within a formulation include those that are substantially free from reacting with functional groups on the polypeptide. In this regard, both reducing and non-reducing sugars can be used as an excipient in a formulation of the invention. However, because reducing sugars contain a hemiacetal group they can react and form adducts or other modifications with amino groups on amino acid side chains of polypeptides (i.e., glycosylation). Similarly, excipients such as citrate, succinate or histidine also can form adducts with amino acid side chains. Given the teachings and guidance provided herein, those skilled in the art will known that greater retention of stability for a given polypeptide can be achieved by choosing a non-reducing sugar over a reducing sugar or over other amino acid-reactive excipients such as those exemplified above.

Optimal excipients also are chosen to enhance or provide stabilization with reference to the mode of administration for an aqueous formulation of the invention. For example, parenteral routes of intravenous (IV), subcutaneous (SC) or intramuscular (IM) administration can be more safe and efficacious when all components of the formulation maintain physical and chemical stability during manufacture, storage and administration. Those skilled in the art will know to employ one or more excipients that maintain maximal stability of the active form of a polypeptide given, for example, a particular manufacturing or storage condition or a particular mode of administration. The excipients exemplified herein for use in a formulation exhibit these and other characteristics.

The amount or concentration of excipient to use in a formulation of the invention will vary depending on, for example, the amount of panitumumab included in the formulation, the amount of other excipients included in the desired formulation, whether a diluent is desired or needed, the amount or volume of other components of the formulation, the total amount of components within a formulation, the specific activity of the polypeptide and the desired tonicity or osmolality to be achieved. Specific examples for excipient concentrations are exemplified further below. Further, different types of excipients can be combined into a single formulation. Accordingly, a formulation of the invention can contain a single excipient, two, three or four or more different types of excipients. Combinations of excipients can be particularly useful in conjunction with a formulation that contains two or more different polypeptides. The excipients can exhibit similar or different chemical properties.

Given the teachings and guidance provided herein, those skilled in the art will know what amount or range of excipient can be included in any particular formulation to achieve a formulation of the invention that promotes retention in stability panitumumab. For example, the amount and type of a salt to be included in a formulation of the invention can be selected based on to the desired osmolality (i.e., isotonic, hypotonic or hypertonic) of the final solution as well as the amounts and osmolality of other components to be included in the formulation. Similarly, by exemplification with reference to the type of polyol or sugar included in a formulation, the amount of such an excipient will depend on its osmolality. Inclusion of about 5% sorbitol can achieve isotonicity while about 9% of a sucrose excipient is needed to achieve isotonicity. Selection of the amount or range of concentrations of one or more excipients that can be included within a formulation of the invention has been exemplified above by reference to salts, polyols and sugars. However, those skilled in the art will understand that the considerations described herein and further exemplified by reference to specific excipients are equally applicable to all types and combinations of excipients including, for example, salts, amino acids, other tonicity agents, surfactants, stabilizers, bulking agents, cryoprotectants, lyoprotectants, anti-oxidants, metal ions, chelating agents and/or preservatives.

Excipients can be included in a formulation of the invention at concentration ranges generally between about 1-40% (w/v), between about 5-35% (w/v), between about 10-30% (w/v), between about 15-25% (w/v) or about 20% (w/v). Concentrations as high as about 45% (w/y), 50% (w/v) or more than 50% (w/v) in certain instances also can be employed in the formulations of the invention. For example, in some instances, it can be desirable to include concentrations up to 60% (w/v) or 75% (w/v) to produce a hypertonic formulation of the invention. Such hypertonic solutions can be diluted to produce an isotonic formulation prior to use if desired. Other useful concentration ranges include between about 1-20%, particularly between about 2-18% (w/v), more particularly between about 4-16% (w/v), even more particularly between about 6-14% (w/v) or between about 8-12% (w/v) or about 10% (w/v). Excipient concentrations and/or amounts less than, greater than or in between these ranges also can be used in a formulation of the invention. For example, one or more excipients can be included in a formulation which constitute less than about 1% (w/v). Similarly, a formulation can contain a concentration of one or more excipients greater than about 40% (w/v). Accordingly, a formulation of the invention can be produced that contains essentially any desired concentration or amount of one or more excipients including, for example, 1, 2, 3, 4, 5, 6, 7, 8, 9, 10, 11, 12, 13, 14, 15, 16, 17, 18, 19 or 20% (w/v) or more. An example is provided below for a formulation of a polypeptide having about 10.0 % excipient

Various excipients useful in a formulation of the invention have been described previously. In the specific formulations described in the Example, exemplified excipients include glycerol, sucrose, trehalose and/or sorbitol, which is employed as a stabilizer. Another excipient exemplified in the formulations described in the Example is polysorbate 80, which is employed in liquid formulations compared to bulk formulations for storage. Other excipients useful in either a liquid or lyophilized formulation of the invention include, for example, fucose, cellobiose, maltotriose, melibiose, octulose, ribose, xylitol, arginine, histidine, glycine, alanine, methionine, glutamic acid, lysine, imidazole, glycylglycine, mannosylglycerate, Triton X-100, Pluoronic F-127, cellulose, cyclodextrin, dextran (10, 40 and/or 70 kD), polydextrose, maltodextrin, ficoll, gelatin, hydroxypropylmeth, sodium phosphate, potassium phosphate, ZnCl₂, zinc, zinc oxide, sodium citrate, trisodium citrate, tromethamine, copper, fibronectin, heparin, human serum albumin, protamine, glycerin, glycerol, EDTA, metacresol, benzyl alcohol and phenol. Excipients such as these as well as others known in the art can be found described in, for example, Wang W., *supra,* (1999) and Wang W., *supra,* (2000).

A buffer component of a formulation of the invention also can include one or more surfactants as an excipient. As described previously, one role of surfactants in a formulation of the invention is to prevent or minimize aggregation and/or adsorption such as surface-induced degradation. At sufficient concentrations, generally about the surfactant's critical micellar concentration, a surface layer of surfactant molecules serve to prevent protein molecules from adsorbing at the interface. Thereby, surface-induced degradation is minimized. Surfactant, their use, formulation and characteristics for formulations are well known in the art and can be found described in, for example, Randolph and Jones, *supra,* (2002).

Optimal surfactants to include in a formulation of the invention can be chosen, for example, to enhance or promote retention in stability of panitumumab by preventing or reducing aggregation and/or adsorption. For example, sorbitan fatty acid esters such as the polysorbates are surfactants exhibiting with a wide range of hydrophilic and emulsifying characteristics. They can be used individually or in combination with other surfactants to cover a wide range of stabilization needs. Such characteristics are particularly suitable for use with polypeptides because they can be tailored to cover the wide range of hydrophobic and hydrophilic characteristics of polypeptides. Considerations for selecting a surfactant include those described previously with reference to excipients in general as well as the hydrophobic character and critical micellar concentration of the surfactant The surfactants exemplified herein, as well as many others well known in the art can be used in a formulation of the invention.

Surfactant concentration ranges for a formulation of the invention include those described previously with reference to excipients in general with particularly useful concentrations being less than about 1% (w/v). In this regard, surfactant concentrations generally can be used at ranges between about 0.001-0.10 % (w/v), particularly between about 0.002-0.05% (w/v), more particularly between about 0.003-0.01% (w/v), even more particularly between about 0.004-0.008% (w/v) or between about 0.005-0.006% (w/v). Surfactant concentrations and/or amounts less than, greater than or in between these ranges also can be used in a formulation of the invention. For example, one or more surfactants can be included in a formulation which constitute less than about 0.001% (w/v). Similarly, a formulation can contain a concentration of one or more surfactants greater than about 0.10% (w/v). Accordingly, a formulation of the invention can be produced that contains essentially any desired concentration or amount of one or more surfactants including, for example, 0.001, 0.002, 0.003, 0.004, 0.005, 0.006, 0.007, 0.008, 0.009, 0.010, 0.02, 0.03, 0.04, 0.05, 0.06, 0.07, 0.08, 0.09 or 0.10% (w/v) or more.

Various surfactants useful as an excipient in a formulation of the invention have been described previously. Other surfactants useful in either a liquid or lyophilized formulation of the invention include, for example, sugar esters such as esters lauric acid (C12), palmitic acid (C16), stearic acid (C18), macrogol cetostearyl ethers, macrogol lauryl ethers, macrogol oleyl ether, macrogol oleate, macrogol stearate, macrogol glycerol ricinoleate, macrogol glycerol hydroxystearate; alkyl polyglucosides such as octyl glucoside and decyl maltoside; fatty alcohols such as cetyl alcohol and oleyl alcohol, and cocamides such as cocamide MEA, DEA, TEA, other non-ionic surfactants and other ionic surfactants.

Therefore, the invention provides a formulation that includes an aqueous solution having between about 1-100 mM acetic acid, glutamic acid or succinic acid with a pH between 4.5-5.5 a polyol or sugar between about 1-20%, polysorbate 80 between about 0.001 - 0.010% and an effective amount of panitumumab wherein panitumumab retains at least 80% stability for up to two months in solution. The formulation of the invention also can include about 10 mM of acetic acid, glutamic acid or succinic acid having a pH of about 5.0, about 2.6% glycerol and about 0.004% polysorbate 80. Various other formulation components, combinations of components and concentrations thereof also can be included in a formulation of the invention.

Further disclosed is a formulation having a therapeutic polypeptide as the polypeptide component of the formulation. The therapeutic polypeptide includes an antibody, a functional fragment of an antibody, a peptibody, a hormone, a growth factor or a cell signaling molecule. In a specific, disclosed ambodiment, the antibody is a human antibody. In another specific disclosed embodiment, the antibody is specific for EGFR. In the case of present invention the antibody is panitumumab.

Also included within a formulation as disclosed herein is a wide variety of therapeutic molecules. A therapeutic molecule as disclosed includes, for example, a macromolecule or biopolymer such as a polypeptide, nucleic acid, lipid, carbohydrate employed as an active pharmaceutical ingredient or building block thereof, that can be used in the diagnosis, treatment or prevention of a pathological condition or as a component of a medication. For example, the formulations as disclosed are applicable to, and facilitate retention in stability for, polypeptides, glycopolypeptides, peptidoglycans, DNA such as genomic DNA, cDNA and the like, RNA such as mRNA, RNAi, SNRPS, and the like, carbohydrates contemplated as an active pharmaceutical ingredient which can include monosaccharides, polysaccharides, N-linked sugars, O-linked sugars, leptins and the like, lipids such as phospholipids, glycolipids, fatty acids, polyamines, isoprenoids, amino acids, nucleotides, neurotransmitters and co-factors, as well as many other macromolecules, biopolymers and building blocks thereof, endogenous to mammalian physiological systems, including human. These and other biopharmaceuticals are well known to those skilled in the art and can be included in a formulation as disclosed for use in the diagnosis, treatment or prevention of a pathological condition or as a component of a medication.

Given the teachings and guidance provided herein, those skilled in the art will understand that a formulation as disclosed is equally applicable to all types of therapeutic molecules, including those exemplified above as well as others well known in the art. Given the teachings and guidance provided herein, those skilled in the art also will understand that the selection of, for example, type(s) or and/or amount(s) of one or more excipients, surfactants and/or optional components can be made based on the chemical and functional compatibility with the therapeutic molecule to be formulated and/or the mode of administration as well as other chemical, functional, physiological and/or medical factors well known in the art. For example, as described previously, non-reducing sugars exhibit favorable excipient properties when used with polypeptide therapeutics compared to reducing sugars. Accordingly, the formulations as disclosed are exemplified further below with reference to polypeptide therapeutics. However, the range of applicability, chemical and physical properties, considerations and methodology applied to polypeptide therapeutics are similarly applicable to therapeutic molecules other than polypeptide therapeutics.

Exemplary types of polypeptides applicable for use in a formulation as disclosed include all types of therapeutic polypeptides including, for example, the immunoglobulin superfamily of polypeptides, growth factors, cytokines, cell signaling molecules and hormones. Exemplary polypeptides applicable for use in a formulation of the invention include all therapeutic polypeptides including, for example, antibodies and functional fragments thereof, interleukins, G-CSF, GM-CSF, kinases, TNF and TNFR ligands including Fhm, cyclins, erythropoietin, nerve growth factors (NGF), developmentally regulated nerve growth factor VGF, neurotrophic factors, neurotrophic factor NNT-1, Eph receptor, Eph receptor ligands; Eph-like receptor, Eph-like receptor ligands, inhibitors of apoptosis proteins (IAP), Thy-1 specific protein, Hek ligand (hek-L), Elk receptor and Elk receptor ligands, STATs, collagenase inhibitor, osteoprotegerin (OPG), APRIL/G70, AGP-3/BLYS, BCMA, TACI, Her-2/neu, Apolipoprotein polyeptides, integrins, tissue inhibitor of metalloproteinases, C3b/C4b complement receptor, SHC binding protein, DKR polypeptides, extracellular matrix polypeptides, antibodies to the above therapeutic polypeptides and antibody functional fragments thereof, antibodies to receptors for the above therapeutic polypeptides and antibody functional fragments thereof, functional polypeptide fragments thereof, fusion polypeptides, chimeric polypeptides and the like.

Specific examples of commercially available pharmaceuticals applicable for use in a formulation as disclosed include, for example, ENBREL (Etanercept; a CHO expressed dimeric fusion protein ((Amgen, Inc.)); EPOGEN (Epoetin alfa; a mammalian cell expressed glycoprotein (Amgen, Inc.)); INFERGEN® (Interferon alfacon-1; an *E. Coli* expressed recombinant protein (Amgen, Inc.)); KINERET® (anakinra; an *E*.*coli* expressed recombinant, nonglycosylated form of the human interleukin-1 receptor antagonist (IL-1 Ra) (Amgen, Inc.)); ARANESP (darbepoetin alfa; a CHO expressed recombinant human erythropoiesis stimulating protein (Amgen, Inc.)); NEULASTA (pegfilgrastim; covalent conjugate of recombinant methionyl human G-CSF and 20kD PEG (Amgen, Inc.)); NEUPOGEN (Filgrastim; an *E. coli* expressed human granulocyte colony-stimulating factor (G-CSF) (Amgen, Inc.)), and STEMGEN (Ancestim, stem cell factor; an *E*.*Coli* expressed recombinant human protein (Amgen, Inc.)). These and all other commercially available pharmaceuticals can be, for example, reformulated in a formulation as disclosed at the time of production, prior to use and/or prior to short or long term storage.

Specific examples of antibodies, in particular antibodies specific to EGFR applicable, for use as a therapeutic antibody in a formulation as disclosed include, for example, panitumumab (Amgen, Inc.); according to the present invention cetuximab (Erbitux^{Tm}; Imclone Systems, New York City); IMC-11F8 (Imclone Systems); Humax-EGFR (Genmab, Copenhagen, Denmark); matuzumab (EMD-7200; Merck KGaA, Darmstadt, Germany), and nimotuzumab (TheraClM hR3; YM Biosciences, Mississauga, Ontario, Canada). All of the above antibodies are well known in the art. For example, panitumumab is commercially available from Amgen and is the subject matter of the human anti-EGFR antibody described in U.S. Patent No. 6,235,883. IMC-11F8 is the subject matter of U.S. Patent No. 7,060,808 and Humax-EGFR is the subject matter of U.S. Patent Publications 20030091561 and 20030194403.

By further illustration of the range of therapeutic molecule applicability of a formulation of as disclosed, described further below are exemplary types of antibodies and functional fragments thereof, that can be employed as a therapeutic polypeptide in a formulation of the invention. As described previously, the chemical and physical properties, formulation considerations and methodology applicable to antibodies and functional fragments thereof, are similarly applicable to biopharmaceuticals including other polypeptide biopharmaceuticals.

Target-specific monoclonal antibodies for use as a polypeptide as disclosed, or functional fragments thereof, can be produced in any of the various antibody forms and/or can be altered or modified in any of the various ways as described previously while still maintaining their specific target binding activity. Any of such antibody forms, alterations or modifications, including combinations thereof, of a target-specific monoclonal antibody, or functional fragment thereof, is included within the disclosure as a polypeptide. Any of such various antibody forms, alterations or modifications of a target-specific monoclonal antibody for use as a polypeptide of the invention, or a functional fragment thereof, can similarly be used in the methods, compositions and/or articles of manufacture of the disclosure as they are described herein. For example, target-specific monoclonal antibodies of the invention, or functional fragments thereof, include target-specific grafted, humanized, Fd, Fv, Fab, F(ab)₂, scFv and peptibody monoclonal antibodies as well as all other forms, alterations and/or modifications described previously, and including other forms well known to those skilled in the art.

Methods for producing hybridomas and screening for target-specific monoclonal antibodies using hybridoma technology are routine and well known in the art. For example, mice can be immunized with a target molecule such as a polypeptide and once an immune response is detected, e.g., antibodies specific for the target molecule are detected in the mouse serum, the mouse spleen is harvested and splenocytes isolated. The splenocytes are then fused by well known methods to any suitable myeloma cells, for example, cells from cell line SP20 available from the ATCC. Hybridomas are selected and cloned by limited dilution. The hybridoma clones are then assayed by methods known in the art for cells that secrete antibodies capable of binding a target molecule. Ascites fluid, which generally contains high levels of antibodies, can be generated by immunizing mice with positive hybridoma clones.

Additionally, recombinant expression in prokaryotic or eukaryotic hosts can be used to generate target-specific monoclonal antibodies. Recombinant expression can be utilized to produce single target-specific monoclonal antibody species, or functional fragments thereof. Alternatively, recombinant expression can be utilized to produce diverse libraries of heavy and light, or variable heavy and variable light chain combinations, and then screened for a monoclonal antibody, or functional fragment thereof, exhibiting specific binding activity to the target molecule. For example, heavy and light chains, variable heavy and light chain domains, or functional fragments thereof, can be co-expressed from nucleic acids encoding target-specific monoclonal antibodies using methods well known in the art to produce specific monoclonal antibody species. Libraries can be produced using methods well known in art from co-expressed populations of nucleic acids encoding heavy and light chains, variable heavy and light chain domains, or functional fragments thereof, and screened by affinity binding to the target molecule for identification of target-specific monoclonal antibodies. Such methods can be found described in, for example, Antibody Engineering: A Practical Guide, C.A.K. Borrebaeck, Ed., supra*;* Huse et al., Science 246:1275-81 (1989); Barbas et al., Proc. Natl. Acad. Sci. USA 88:7978-82 (1991); Kang et al., Proc. Natl. Acad. Sci. USA 88:4363-66 (1991); Plückthun and Skerra, *supra*; Felici et al., J. Mol. Biol. 222:301-310 (1991); Lerner et al., Science 258:1313-14 (1992), and in U.S. Patent No. 5,427,908.

Cloning of encoding nucleic acids can be accomplished using methods well known to those skilled in the art. Similarly, cloning of heavy and/or light chain repertoires of encoding nucleic acid, including V_{H} and/or V_{L} encoding nucleic acids also can be accomplished by methods well known to those skilled in the art. Such methods include, for example, expression cloning, hybridization screening with a complementary probe, polymerase chain reaction (PCR) using a complementary pair of primers or ligase chain reaction (LCR) using a complementary primer, reverse transcriptase PCR (RT-PCR) and the like. Such methods can be found described in, for example, Sambrook et al., Molecular Cloning: A Laboratory Manual, Third Ed., Cold Spring Harbor Laboratory, New York (2001) and Ansubel et al., Current Protocols in Molecular Biology, John Wiley and Sons, Baltimore, MD (1999).

Encoding nucleic acids also can be obtained from any of various public databases including whole genome databases such as those operated by The National Center for Biotechnology Information (NCB1) of the National Institutes of Health (NIH). A particularly useful method of isolating either a single encoding nucleic or a repertoire of encoding nucleic acids for heavy and/or light chains, or functional fragments thereof, can be accomplished without specific knowledge of the coding region portion because primers are available or can be readily designed using conserved portions of antibody variable or constant region portions. For example, a repertoire of encoding nucleic acids can be cloned using a plurality of degenerate primers to such regions together with PCR. Such methods are well known in the art and can be found described in, for example, Huse et al., *supra,* and Antibody Engineering: A Practical Guide, C.A.K. Borrebaeck, Ed., supra*.* Any of the above methods as well as others known in the art, including combinations thereof, can be used to generate a target-specific monoclonal antibody for use as a polypeptide of the invention.

Therefore, the disclosure provides a formulation having an antibody, a functional fragment of an antibody as a therapeutic polypeptide. The therapeutic polypeptide can include a monoclonal antibody, Fd, Fv, Fab, F(ab'), F(ab)₂, F(ab')₂, single chain Fv (scFv), chimeric antibodies, diabodies, triabodies, tetrabodies, minibody or peptibody.

Concentrations of a polypeptide to be included in a formulation of the disclosure and the invention will vary, for example, depending on the activity of the polypeptide, the indication to be treated, mode of administration, the treatment regime and whether the formulation is intended for long term storage in either liquid or lyophilized form. Those skilled in the art will know what concentrations to use given these well known considerations and the state of the art in the pharmaceutical sciences. For example, there are more than 80 polypeptides approved for therapeutic use in the United States for a wide range of medical indications, modes of administration and treatment regimes. These approved polypeptides are exemplary of the range of polypeptide concentrations that can be used in a formulation of the invention.

Generally, a polypeptide including, for example, a therapeutic polypeptide in the case of the invention panitumumab, will be included in a formulation of the invention at a concentration from between about 1-200 mg/ml, about 10-200 mg/ml, about 20-180 mg/ml, particularly between about 30-160 mg/ml, more particularly between about 40-120 mg/ml, even more particularly between about 50-100 mg/ml or about 60-80 mg/ml. Polypeptide concentrations and/or amounts less than, greater than or in between these ranges also can be used in a formulation of the invention. For example, one or more polypeptides can be included in a formulation which constitute less than about 1.0 mg/ml. Similarly, a formulation can contain a concentration of one or more polypeptides greater than about 200 mg/ml, particularly when formulated for storage. Accordingly, a formulation of the invention can be produced that contains essentially any desired concentration or amount of one or more polypeptides including, for example, 1, 2, 3, 4, 5, 6, 7, 8, 9, 10, 11, 12, 13, 14, 15, 16, 17, 18, 19, 20, 25, 30, 35, 40, 45, 50, 55; 60, 65, 70, 75, 80, 85, 90, 95, 100, 110, 120, 130, 140, 150, 160, 170, 180, 190 or 200 mg./ml or more. Exemplified in the Example below is a formulation for a therapeutic polypeptide having a concentration of about 10 mg/ml.

A formulation of the invention also can include combinations of polypeptides in the formulation. For example, a formulation of the invention can include a single polypeptide for treatment of one or more conditions. A formulation of the invention also can include two or more different polypeptides. Use of multiple polypeptides in a formulation of the invention can be directed to, for example, the same or different indications. Similarly, multiple polypeptides can be used in a formulation of the invention to treat, for example, both a pathological condition and one or more side effects caused by the primary treatment. Multiple polypeptides also can be included in a formulation of the invention to accomplish different medical purposes including, for example, simultaneous treatment and monitoring of the progression of the pathological condition. Multiple, concurrent therapies such as those exemplified above as well as other combinations well known in the art are particularly useful for patient compliance because a single formulation can be sufficient for some or all suggested treatments and/or diagnosis. Those skilled in the art will know those polypeptides that can be admixed for a wide range of combination therapies. Similarly, a formulation of the invention also can be used with small molecule pharmaceuticals and combinations of one or more polypeptides together with one or more small molecule pharmaceuticals. Therefore, the invention provides for a formulation of the invention containing 1, 2, 3, 4, 5 or 6 or more different polypeptides as well as for one or more polypeptides combined with one or more small molecule pharmaceuticals.

A formulation of the invention also can include one or more preservatives and/or additives well known in the art. Similarly, a formulation of the invention can further be formulated into any of various know delivery formulations. For example, a formulation of the invention can include lubricating agents, emulsifying agents, suspending agents, preserving agents such as methyl- and propylhydroxy-benzoates, sweetening agents and flavoring agents. Such optional components, their chemical and functional characteristics are well known in the art. Similarly well known in the art are formulations that facilitate rapid, sustained or delayed release of the polypeptide after administration. A formulation of the invention can be produced to include these or other formulation components well known in the art.

A formulation of the invention also can be produced, for example, in states other than an aqueous liquid. For example, the acetic acid, glutamic acid or succinic acid buffer containing formulations of the invention can be prepared, for example, as a lyophilized formulation.

Once a formulation of the invention is prepared as described herein, stability of the one or more polypeptides contained within the formulation can be assessed using methods well known in the art. Several of such methods are exemplified further below in the Examples and include size exclusion chromatography and particle counting. Any of a variety of functional assays including, for example, binding activity, other biochemical activity and/or physiological activity can be assessed at two or more different time points to determine the stability of the polypeptide in the buffered formulation of the invention.

A formulation of the invention will, in general, be prepared according to pharmaceutical standards and using pharmaceutical grade reagents. Similarly, a formulation of the invention will, in general, be prepared using sterile reagents in a sterile manufacturing environment or sterilized following preparation. Sterile injectable solutions can be prepared using well known procedures in the art including, for example, by incorporating one or more polypeptides in the required amount in an acetic acid, glutamic acid or succinic acid buffer or excipient of the invention with one or a combination of formulation components described herein followed by sterilization microfiltration. In the specific embodiment of sterile powders for the preparation of sterile injectable solutions, particularly useful methods of preparation include, for example, vacuum drying and freeze-drying (lyophilization) as described previously. Such drying methods will yield a powder of the one or more polypeptides together with any additional desired components from a previously sterile-filtered solution thereof.

Administration and dosage regimens can be adjusted to provide an effective amount for an optimum therapeutic response. For example, a single bolus can be administered, several divided doses can be administered over time or the dose can be proportionally reduced or increased as indicated by the exigencies of the therapeutic situation. It can be particularly useful to formulate a formulation of the invention for intravenous, parenteral or subcutaneous injection in a unit dosage form for ease of administration and uniformity of dosage in administering an effective amount of one or more polypeptides. Unit dosing refers to a physically discrete amount of pharmaceutical suited as unitary dosages for the subjects to be treated; each unit contains a predetermined quantity of active polypeptide calculated to produce a desired therapeutic effect.

For further exemplification, an effective amount of a polypeptide such as a therapeutic antibody, particularly panitumumab, can be administered, for example, more than once, at scheduled intervals over a period of time. In certain embodiments, a therapeutic antibody is administered over a period of at least a month or more including, for example, one, two, or three months or longer. For treating chronic conditions, long-term, sustained treatment is generally most effective. Shorter periods of administration can be sufficient when treating acute conditions including, for example, from one to six weeks. In general, a therapeutic antibody or other polypeptide is administered until the patient manifests a medically relevant degree of improvement over baseline for the chosen indicator or indicators.

Depending on the selected polypeptide and indication to be treated, a therapeutically effective amount is sufficient to cause a reduction in at least one symptom of the targeted pathological condition by at least about 5%, 10%, 15%, 20%, 25%, 30%, 35%, 40%, 45%, 50%, 55% or 60% or more, relative to untreated subjects. The ability of a formulation to reduce or inhibit a symptom can be evaluated, for example, in an animal model system predictive of efficacy for the targeted condition in human. Alternatively, the ability of a formulation to reduce or inhibit a symptom can be evaluated, for example, by examining an *in vitro* function or activity of the formulation indicative of *in vivo* therapeutic activity.

Actual dosage levels of one or more polypeptides in a formulation of the invention can be varied so as to obtain an amount of the active polypeptide which is effective to achieve the desired therapeutic response for a particular patient, formulation, and mode of administration, without being toxic to the patient. One skilled in the art would be able to determine administered amounts based on factors such as the subject's size, the severity of the subject's symptoms, and the selected polypeptide and/or route of administration. The selected dosage level can depend, for example, upon a variety of pharmacokinetic factors including the activity of the polypeptide employed, the route of administration, the time of administration, the rate of excretion, the duration of the treatment, other drugs, compounds and/or materials used in combination with the particular compositions employed, the age, sex, weight, condition, general health and prior medical history of the patient being treated, and like factors well known in the medical arts. Particular embodiments of the present invention involve administering a therapeutic polypeptide such as an antibody, or functional fragment thereof, in a formulation of the invention at a dosage of from about 1 ng of antibody per kg of subject's weight per day (1 ng/kg/day) to about 10 mg/kg/day, more particularly from about 500 ng/kg/day to about 5 mg/kg/day, and even more particularly from about 5 µg/kg/day to about 2 mg/kg/day, to a subject.

A physician or veterinarian having skill in the art can readily determine and prescribe the effective amount of the required pharmaceutical formulation. For example, the physician or veterinarian can initiate doses of a formulation of the invention at levels lower than that required in order to achieve the desired therapeutic effect and gradually increase the dosage until the desired effect is achieved. In general, a suitable daily dose of a formulation of the invention will be that amount of the polypeptide which is the lowest dose effective to produce a therapeutic effect. Such an effective amount will generally depend upon the factors described previously. It is particularly useful that administration be intravenous, intramuscular, intraperitoneal, or subcutaneous. If desired, the effective daily dose to achieve an effective amount of a formulation can be administered as two, three, four, five, six or more sub-doses administered separately at appropriate intervals throughout the day, optionally, in unit dosing amounts.

A formulation of the invention can be administered, for example, with medical devices known in the art. For example, in a particularly useful embodiment, a formulation of the invention can be administered with a needleless hypodermic injection device, such as the devices described in U.S. Pat. Nos. 5,399,163; 5,383,851; 5,312,335; 5,064,413; 4,941,880; 4,790,824; or 4,596,556. Examples of well-known implants and modules useful in the present invention include: U.S. Pat. No. 4,487,603, which describes an implantable micro-infusion pump for dispensing medication at a controlled rate; U.S. Pat. No. 4,486,194, which describes a therapeutic device for administering medicants through the skin; U.S. Pat. No. 4,447,233, which describes a medication infusion pump for delivering medication at a precise infusion rate; U.S. Pat. No. 4,447,224, which describes a variable flow implantable infusion apparatus for continuous drug delivery; U.S. Pat. No. 4,439,196, which describes an osmotic drug delivery system having multi-chamber compartments, and U.S. Pat. No. 4,475,196, which describes an osmotic drug delivery system. Many other such implants, delivery systems, and modules are known to those skilled in the art.

In certain specific embodiments, panitumumab for use in a formulation of the invention can additionally be formulated to facilitate selective distribution *in vivo.* For example, the blood-brain barrier (BBB) excludes many highly hydrophilic compounds. To facilitate crossing of the BBB if desired, a formulation can additionally include, for example, liposomes for encapsulation of one or more polypeptides. For methods of manufacturing liposomes, see, for example, U.S. Pat. Nos. 4,522,811; 5,374,548; and 5,399,331. The liposomes can further contain one or more moieties which are selectively transported into specific cells or organs, thus enhancing targeted delivery of a selected polypeptide (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180) or surfactant protein A receptor (Briscoe et al. (1995) Am. J. Physiol. 1233:134).

Therefore, the disclosure additionally provides a method of preparing a formulation. The method includes combining an aqueous solution having an acetic acid, glutamic acid or succinic acid buffer having a pH from about 4.5 to about 7.0 and an excipient selected from a sugar or polyol with an effective amount of a therapeutic polypeptide including, for example, an EGFR specific antibody. The EGFR specific antibody can be, for example, panitumumab. One or more of the formulation components described herein can be combined with one or more effective amounts of a polypeptide to produce a wide range of formulations of the invention.

Additionally disclosed is a container containing a formulation including an aqueous solution having between about 1-10 mM acetic acid, glutamic acid or succinic acid buffer with a pH from about 4.5 to about 7.0, glycerol or sorbitol between about 1-10%, polysorbate 80 between about 0.001-0.010% and an effective amount of a therapeutic antibody, including, for example, an EGFR specific antibody or panitumumab. Briefly, with respect to compositions, kits and/or medicaments disclosed herein, the combined effective amounts of one or more polypeptides within a formulation of the invention can be included within a single container or container means, or included within distinct containers or container means. Imaging components can optionally be included and the packaging also cab include written or web-accessible instructions for using the formulation. A container or container means includes, for example, a vial, bottle, syringe or any of a variety of formats well known in the art for multi-dispenser packaging.

It is understood that modifications which do not substantially affect the activity of the various embodiments of this invention are also included within the definition of the invention provided herein. Accordingly, the following examples are intended to illustrate but not limit the present invention.

### EXAMPLE I

### Polypeptide Stability Characterization in Buffered Solutions

This Example describes the characterization of various formulations on the stability of panitumumab. Also described is the characterization of various formulations on the long term stability of panitumumab bulk preparations.

A variety of formulation conditions that stabilize monoclonal IgG2 antibody panitumumab are described below. These formulation conditions include those applicable for administration of the therapeutic polypeptide as well as for the storage, maintenance and/or lot preparation of the therapeutic polypeptide. The formulation conditions of the invention exemplified below confer particularly useful panitumumab stability against aggregation, chemical degradation and particle formation. These conditions were shown to be particularly effective in preventing particle formation which allows elimination of any need for in-line filter for intravenous administration.

Briefly, panitumumab was found to be stable at a pH ranging from about 5.0 to 7.0. Optimal stability was observed at a pH of 5.0 with respect to aggregation and particle formation. Formulations with a pH of 5.0 also were the clearest (ie, most transparent) liquid solution, indicating less aggregation. Particularly useful buffer systems included acetic acid, L-glutamic acid and succinic acid. All three of these buffer systems worked well near a pH of about 5.0 (e.g., from about 4.8 to about 5.2). Among these buffer systems, L-glutamic acid was observed to be equally effective or better than acetic acid for panitumumab stability. Particularly useful excipients for panitumumab included glycerol, sucrose, trehalose and sorbitol. All showed effective stabilizing properties with respect to aggregation and/or particle formation. Optimal excipients, included glycerol and sucrose.

The results set forth below show a variety of formulations that maintain, augment or optimize panitumumab stability. In certain specific formulations, particularly useful liquid formulations for panitumumab included 10mM acetic acid, 2.6% glycerol, 0.004% polysorbate 80 at pH 5.0 and 10 mM L-glutamic acid, 2.6% glycerol, 0.004% polysorbate 80 at pH 5.0. In other specific formulations, particularly useful long-term formulations for, for example, frozen storage, maintenance and/or lot preparation such as bulk substance preparation, included 10 mM acetic acid, 2.6% glycerol at pH 5.0 and 10 mM L-glutamic acid, 2.6% glycerol at pH 5.0 when the panitumumab formulation is maintained at -30C or below. Glycerol, sucrose and trehalose were further found to be particularly useful excipients that protected panitumumab from freeze-thaw-induced aggregation and particle formation.

The studies described herein were directed to the characterization and selection of formulations that augment retention in stability of panitumumab. Based on preliminary analysis, three buffer systems were chosen for characterizing stable liquid and frozen formulations for panitumumab. These buffer systems were acetic acid, glutamic acid and succinic acid. The characterization of formulations derived from these buffer systems is exemplified below.

One initial characterization was the visual appearance of panitumumab in various acetic acid buffer formulations. Briefly, the seven formulations listed below were assessed at different pH values.
1. pH 5.0 : 20mg/ml panitumumab, 5mM Acetate, 5mM Phosphate, 5% Sorbitol, pH 5.0
2. pH 5.5 : 20mg/ml panitumumab, 5mM Acetate, 5mM Phosphate, 5% Sorbitol, pH 5.5
3. pH 6.0 : 20mg/ml panitumumab, 5mM Acetate, 5mM Phosphate, 5% Sorbitol, pH 6.0
4. pH 6.5 : 20mg/ml panitumumab, 5mM Acetate, 5mM Phosphate, 5% Sorbitol, pH 6.5
5. pH 7.0 : 20mg/ml panitumumab, 5mM Acetate, 5mM Phosphate, 5% Sorbitol, pH 7.0
6. pH 7.5 : 20mg/ml panitumumab, 5mM Acetate, 5mM Phosphate, 5% Sorbitol, pH 7.5
7. A58N : 20mg/ml panitumumab, 50mM Acetate, 100mM NaCl, pH 5.8 (control)

Figure 1 shows the visual appearance of panitumumab formulated at pH values ranging from 5.0 to 7.5. The results indicate that the protein solution is clearer and more transparent at lower pH values. In comparison, formulations became more turbid at higher pH values.

Accelerated stability studies were performed to characterize the stability of panitumumab under different pH conditions. Briefly, accelerated stability studies performed at a particular pH and at, for example, 37 C in glass vials. Samples were dialyzed into the respective formulations to be tested and sterile filtered into sterile containers. Approximately 2-mL quantities of each formulated sample were placed in sterile 3-mL glass vials in a sterile hood and stoppered. Samples designated for freezing were placed in sterile polypropylene eppindorf tubes. All vials were labeled and crimped followed by placement into boxes specified for storage at -80 C, 2-8 C, and 37 C conditions. Samples were removed and analyzed at designated time points. Size exclusion chromatography (SEC) was used as one of the analytical methods. A TosoHaas G3000SWx1 dual column in tandem was used to carry out the analysis using a mobile phase consisting of 100 mM phosphate (pH 7), 150 mM NaCl

Different forms of the samples could be quantitatively evaluated and separated based on their hydrodynamic volume. Exemplary results are illustrated in Figure 2 and show the percent monomer of panitumumab stored at 37C for up to 2 months. Higher monomer losses were observed at higher pH conditions. The formulations exemplified in Figure 2 at each time point were:
1. EGF_20pH 5.0: 20mg/m1 panitumumab, 5mM Acetate, 5mM Phosphate, 5% Sorbitol, pH 5.0
2. EGF_20pH 5.5: 20mg/ml panitumumab, 5mM Acetate, 5mM Phosphate, 5% Sorbitol, pH 5.5
3. EGF_20pH 6.0: 20mg/ml panitumumab, 5mM Acetate, 5mM Phosphate, 5% Sorbitol, pH 6.0
4. EGF_20pH 6.5: 20mg/ml panitumumab, 5mM Acetate, 5mM Phosphate, 5% Sorbitol, pH 6.5
5. EGF_20pH 7.0 : 20mg/ml panitumumab, 5mM Acetate, 5mM Phosphate, 5% Sorbitol, pH 7.0
6. EGF_20pH 7.5: 20mg/ml panitumumab, 5mM Acetate, 5mM Phosphate, 5% Sorbitol, pH 7.5
7. EGF_20pH A58N: 20mg/ml panitumumab, 50mM Acetate, 100mM NaCl, pH 5.8 (control)

Changes in charge variance of the protein solution was also determined by cation exchange chromatography (CEX) for the samples stored above for up to 2 months. Briefly, panitumumab was evaluated using cation exchange procedures known in the art. This method separated predominant C-terminal lysine isoforms based on protein surface charge differences using a linear salt gradient at pH 6.2 and a Dionex weak-cation exchange column (WCX-10; Sunnyvale, CA) and also acidic modification of some amino acids represented by deamidation.

CEX data for the seven formulations shown below having different pH conditions and being stored for up to 2 months at 37C are presented in Figure 3. The result shows that the percentage of acid variants (represented by peak 0, which indicates deamidation products) is minimal at acidic pH (5.0 and 5.5).
1. EGF_20pH 5.0: 20mg/ml panitumumab, 5mM Acetate, 5mM Phosphate, 5% Sorbitol, pH 5.0
2. EGF_20pH 5.5: 20mg/ml panitumumab, 5mM Acetate, 5mM Phosphate, 5% Sorbitol, pH 5.5
3. EGF_20pH 6.0: 20mg/ml panitumumab, 5mM Acetate, 5mM Phosphate, 5% Sorbitol, pH 6.0
4. EGF_20pH 6.5: 20mg/ml panitumumab, 5mM Acetate, 5mM Phosphate, 5% Sorbitol, pH 6.5
5. EGF_20pH 7.0: 20mg/ml panitumumab, 5mM Acetate, 5mM Phosphate, 5% Sorbitol, pH 7.0
6. EGF_20pH 7.5: 20mg/ml panitumumab, 5mM Acetate, 5mM Phosphate, 5% Sorbitol, pH 7.5
7. EGF_20pH A58N: 20mg/ml panitumumab, 50mM Acetate, 100mM NaCl, pH 5.8 (control)

One characteristic relating to monoclonal antibodies and other polypeptides is the occurrence of subvisible insoluble particles. In this context, a polypeptide particle refers to, for example, a fragment or aggregate of the polypeptide and can be soluble and/or insoluble. Additionally, particles can be made up of matter that is foreign (i.e., shards of glass, lint, small pieces of rubber stopper) and not necessarily composed of the polypeptide. Soluble aggregtes/particles can be evaluated using methods such as SEC, for example. Particles that are insoluble can be evaluated using such methods as liquid particle counting or light obscuration approach such as HIAC, for example. Coarse particles are generally classified as particles having sizes greater than 1.0 µm and those considered fine particles are smaller in size. Using the LD-400 laser system with the HIAC instrument (Geneva, Switzerland), particle sizes between 2 and 400 µm can be measured.

Formation of insoluble particles was assessed for seven exemplary formulations assessing different pH conditions using liquid particle counting. For reference, these formulations as they are denoted in Figure 4 were:
1. pH 5.0 : 20mg/ml panitumumab in 5mM Acetate, 5mM Phosphate, 5% Sorbitol, pH 5.0
2. pH 5.5 : 20mg/ml panitumumab in 5mM Acetate, 5mM Phosphate, 5% Sorbitol, pH 5.5
3. pH 6.0 : 20mg/ml panitumumab in 5mM Acetate, 5mM Phosphate, 5% Sorbitol, pH 6.0
4. pH 6.5 : 20mg/ml panitumumab in 5mM Acetate, 5mM Phosphate, 5% Sorbitol, pH 6.5
5. pH 7.0 : 20mg/ml panitumumab in 5mM Acetate, 5mM Phosphate, 5% Sorbitol, pH 7.0
6. pH 7.5 : 20mg/ml panitumumab in 5mM Acetate, 5mM Phosphate, 5% Sorbitol, pH 7.5
7. A58N: 20mg/ml panitumumab in 50mM Acetate, 100mM NaCl, pH 5.8 (control)

The HIAC particle counter instrument was equipped with PharmSpec software version 1.4, required to measure the 10 µm and 25 µm particles present in a given Emab sample. The employed methods followed procedures complying with USP requirements of particle assessment and quality. Filtered water (0.22 micron) was drawn through a stainless steal tube using 1.0 mL volumes and flushed approximately 10 times between sample measurements. Duke scientific EZY-CAL liquid particle 10 µm size standard was used to verify proper calibration of the instrument. Both sample and standard measurements were taken with a volume of 0.2 mL, drawn 4 times, discarding the first run and averaging the last two or three. The samples were drawn from their original vials, with a slight swirl given to each sample prior to measurement to ensure uniform mixing of the solution. The standard was vigorously shaken prior to measurement

The results of the HIAC particle counts counts of panitumumab formulated at various pHs after vortexing for 15 minutes at 4C are shown in Figure 4. Particles ranging from 5 µm to 25 µm in size were counted. The results show that all samples formulated at pH from 5.0 to 7.0 exhibited lower particle counts than those formulated at pH 7.5. Particle counts formulated in the buffer containing sodium chloride (A58N) were significantly higher than those formulated in the sorbitol buffers,

Based on the above exemplary results, a pH of 5.0 was selected for characterization of further formulations as described below.

Size exclusion chromatography was employed as described above to assess the stability of panitumumab formulated in acetic acid buffer, succinic acid buffer or glutamic acid buffer following storage at 37C for up to 4 months. The formulations are set forth below as follows:
1. A_2.6%Glycerol_pH5_T80: 20 mg/mL panitumumab in 10mM acetic acid acid, 2.6% glycerol, pH 5.0, 0.004% Tween 80.
2. Succ_2.6%Glycerol_pH5_T80: 20 mg/mL panitumumab in 10mM succinic acid acid, 2.6% glycerol, pH 5.0, 0.004% Tween 80.
3. Gluta_2.6Glycerol_pH5_T80: 20 mg/mL panitumumab in 10mM L-glutamic acid, 2.6% glycerol, pH 5.0, 0.004% Tween 80.

The results of this study are shown in Figure 5. Briefly, similar monomer content was observed for all buffer systems. The succinic acid containing formulations revealed a slightly lower monomer content and the glutamic acid containing formulation maintained the exhibited the most amount of monomer after 4 months storage at 37C.

Stability of panitumumab formulations in either acetic acid, glutamic acid or succinic acid also were assessed in for longer periods of time and for different temperatures as described below. Briefly, cation exchange chromatography as described previously was employed to assess panitumumab stability in these buffer systems following incubation at 29C for up to 6 months. As shown in Figure 6, the formulations included:
1. A_2.6%Glycerol_pH5_T80: 20 mg/mL panitumumab in 10mM acetic acid acid, 2.6% glycerol, pH 5.0, 0.004% Tween 80.
2. Succ_2.6%Glycerol_pH5_T80: 20 mg/mL panitumumab in 10mM succinic acid acid, 2.6% glycerol, pH 5.0, 0.004% Tween 80.
3. Gluta_2.6Glycerol_pH5_T80: 20 mg/mL panitumumab in 10mM L-glutamic acid, 2.6% glycerol, pH 5.0, 0.004% Tween 80.panitumumab

The result shown in Figure 6 indicate that the percentage of acid variants (represented by peak 0, which indicates deamidation products) is comparable in all formulations. Minimal variants formation was observed using a glutamic acid buffer system.

Particle formation of panitumumab in either acetic acid, glutamic acid or succinic acid also were assessed using HIAC particle analysis as described previously. Briefly, panitumumab was formulated as set forth below and incubated at 4C for 6 months.
1. Ace2.6glycerolT80pH5.0: 20 mg/mL panitumumab in 10mM acetic acid acid, 2.6% glycerol, pH 5.0, 0.004% Tween 80.
2. Succ2.6glycerolT80pH5.0: 20 mg/mL panitumumab in 10mM succinic acid acid, 2.6% glycerol, pH 5.0, 0.004% Tween 80.
3. Gluta2.6glycerolT80pH5.0: 20 mg/mL panitumumab in 10mM L-glutamic acid, 2.6% glycerol, pH 5.0, 0.004% Tween 80.

The result are shown in Figure 7 and indicate acceptable particle counts in all formulations. As judged by USP guideline, there were very few particles of >10 µm and >25 µm, although particles counts of >2 µm in size in the acetate buffer was observed to be higher than those in either glutamate or succinate buffers.

Figure 8 shows the monomer content as measured by SEC HPLC of various isotonic formulations containing different excipients. SEC HPLC was performed as described previously. The different excipents characterized included sorbitol (S), glycerol (GLY), arginine (ARG), sucrose (SUC) and polysorbate 80 (T80). Complete formulations for are shown below for samples stored for up to 2 years at 4C. The result indicate that panitumumab is stable in sorbitol, glycerol, sucrose and polysorbate 80. Less stability was observed for argine.

| | | | | | |
|---|---|---|---|---|---|
| A5S | 10 mM NaAcetate | 5% Sorbitol | pH 5.0 | | 20 mg/mL |
| A5ST | 10 mM NaAcetate | 5% Sorbitol | pH 5.0 | 0.004% Tween-80 | 20 mg/mL |
| GLY5 | 10 mM NaAcetate | 2.6% Glycerol | pH 5.0 | | 20 mg/mL |
| GLY5T | 10 mM NaAcetate | 2.6% Glycerol | pH 5.0 | 0.004% Tween-80 | 20 mg/mL |
| ARG5 | 10 mM NaAcetate | 2.5% Arginine | pH 5.0 | | 20 mg/mL |
| ARG5T | 10 mM NaAcetate | 2.5% Arginine | pH 5.0 | 0.004% Tween-80 | 20 mg/mL |
| SUC5 | 10 mM NaAcetate | 9.3% Sucrose | pH 5.0 | | 20 mg/mL |
| SUC5T | 10 mM NaAcetate | 9.3% Sucrose | pH 5.0 | 0.004% Tween-80 | 20 mg/mL |
| A58N | 50 mM NaAcetate | 100mM NaCl | pH 5.8 | | 20 mg/mL |
| A58NT | 50 mM NaAcetate | 100mM NaCl | pH 5.8 | 0.004% Tween-80 | 20 mg/mL |

Figure 9 shows particle count measurements for the above different excipients for particle sizes >10 µm using the HIAC method as described previously. The formulations and key for Figure 9 are provided below. The results indicate that panitumumab is stable in a variety of formulations containing sorbitol, glycerol, sucrose and salt either in the presence or absence of polysorbate when stored for 1 year at 4C.

| | | | | | |
|---|---|---|---|---|---|
| A5S | 10 mM NaAcetate | 5% Sorbitol | pH 5.0 | | 20 mg/mL |
| A5ST80 | 10 mM NaAcetate | 5% Sorbitol | pH 5.0 | 0.004% Tween-80 | 20 mg/mL |
| 2.6GLY5 | 10 mM NaAcetate | 2.6% Glycerol | pH 5.0 | | 20 mg/mL |
| 2.6GLY5T80 | 10 mM NaAcetate | 2.6% Glycerol | pH 5.0 | 0.004% Tween-80 | 20 mg/mL |
| ARG5 | 10 mM NaAcetate | 2.5% Arginine | pH 5.0 | | 20 mg/mL |
| ARG5T | 10 mM NaAcetate | 2.5% Arginine | pH 5.0 | 0.004% Tween-80 | 20 mg/mL |
| SUC5 | 10 mM NaAcetate | 9.3% Sucrose | pH 5.0 | | 20 mg/mL |
| SUC5T | 10 mM NaAcetate | 9.3% Sucrose | pH 5.0 | 0.004% Tween-80 | 20 mg/mL |
| A58N | 50 mM NaAcetate | 100mM NaCl | pH 5.8 | | 20 mg/mL |
| A58NT | 50 mM NaAcetate | 100mM NaCl | pH 5.8 | 0.004% Tween-80 | 20 mg/mL |

In addition to the above formulations, a number of additional components and formulations were characterized for panitumumab stability under long-term storage conditions and with respect to freeze-thaw cycles. These characterizations are described further below. All assay methods were performed as described previously.

Briefly, Figure 10 show the monomer percentage of panitumumab analyzed by SE-HPLC in various formulations at a pH ranging from 5.0 to 7.0. Different excipients were included as set forth below for each formulation. The panitumumab samples were stored for up to 3 months at -30C. These formulations were studied in relation to developing a frozen formulation or frozen drug substance or a bulk substance solution.
1. EGF_p5glycerol2.6: 40 mg/mL panitumumab in 10 mM acetate, 2.6% glycerol, pH 5.0
2. EGF_p5glycerol10: 40 mg/mL panitumumab in 10 mM acetate, 10% glycerol, pH 5.0
3. EGF_p5suc9.3: 40 mg/mL panitumumab in 10 mM acetate, 9.3% sucrose, pH 5.0
4. EGF_p5suc20: 40 mg/mL panitumumab in 10 mM acetate, 20% sucrose, pH 5.0
5. EGF_p5tre9.3: 40 mg/mL panitumumab in 10 mM acetate, 9.3% trehalose, pH 5.0
6. EGF_p5tre20: 40 mg/mL panitumumab in 10 mM acetate, 20% trehalose, pH 5.0
7. EGF_p5arg2.5: 40 mg/mL panitumumab in 10 mM acetate, 2.5% arginine, pH 5.0
8. EGF_p5arg10: 40 mg/mL panitumumab in 10 mM acetate, 10% arginine, pH 5.0
9. EGF_p6glycerol2.6: 40 mg/mL panitumumab in 10 mM potassium phosphate, 2.6% glycerol, pH 6.0
10. EGF_p6glycerol10: 40 mg/mL panitumumab in 10 mM potassium phosphate, 10% glycerol, pH 6.0
11. EGF_p6suc9.3: 40 mg/mL panitumumab in 10 mM potassium phosphate, 9.3% sucrose, pH 6.0
12. EGF_p6suc20: 40 mg/mL panitumumab in 10 mM potassium phosphate, 20% sucrose, pH 6.0
13. EGF_p6tre9.3: 40 mg/mL panitumumab in 10 mM potassium phosphate, 9.3% trehalose, pH 6.0
14. EGF_p6tre20: 40 mg/mL panitumumab in 10 mM potassium phosphate, 20% trehalose, pH 6.0
15. EGF_p6arg2.5: 40 mg/mL panitumumab in 10 mM potassium phosphate, 2.5% arginine, pH 6.0
16. EGF_p6arg10: 40 mg/mL panitumumab in 10 mM potassium phosphate, 10% arginine, pH 6.0
17. EGF_p7glycerol2.6: 40 mg/mL panitumumab in 10 mM potassium phosphate, 2.6% glycerol, pH 7.0
18. EGF_p7glycerol10: 40 mg/mL panitumumab in 10 mM potassium phosphate, 10% glycerol, pH 7.0
19. EGF_p7suc9.3: 40 mg/mL panitumumab in 10 mM potassium phosphate, 9.3% sucrose, pH 7.0
20. EGF_p7suc20: 40 mg/mL panitumumab in 10 mM potassium phosphate, 20% sucrose, pH 7.0
21. EGF_p7tre9.3: 40 mg/mL panitumumab in 10 mM potassium phosphate, 9.3% trehalose, pH 7.0
22. EGF_p7tre20: 40 mg/mL panitumumab in 10 mM potassium phosphate, 20% trehalose, pH 7.0
23. EGF_p7arg2.5: 40 mg/mL panitumumab in 10 mM potassium phosphate, 2.5% arginine, pH 7.0
24. EGF_p7arg10: 40 mg/mL panitumumab in 10 mM potassium phosphate, 10% arginine, pH 7.0
25. EGF_A58N: 50 mM acetate, 100 mM sodium chloride, pH 5.8 (control)
26. EGF_A5S: 10 mM acetate, 5% sorbitol, pH 5.0 (control)
26. EGF_H58Suc: 50 mM histidine, 1% sucrose, pH 5.8 (control)

Figure 11 shows the percent monomer of panitumumab analyzed by SE-HPLC as a funtion of pH (5 to 6) and a variety of stabilizers in either acetate or phosphate buffer. The result indicate that when panitumumab is stored at -30C for up to one year the monomer content does not change significantly. The following formulations were characterized:
1. EGF_p5gly2.6: 10mM Acetate, 2.6% Glycerol, pH 5.0
2. EGF_p5gly10: 10mM Acetate, 10% Glycerol, pH 5.0
3. EGF_p5suc9: 10mM Acetate, 9.3% Sucrose, pH 5.0
4. EGF_p5suc20: 10mM Acetate, 20% Sucrose pH 5.0
5. EGF_p5arg2.5: 10mM Acetate, 2.5% Arginine pH 5.0
6. EGF_p5A5S: 10mM Acetate, 5% Sorbitol pH 5.0
7. EGF_p55gly2.6: 10mM Acetate, 2.6% Glycerol pH 5.5
8. EGF_p55gly10: 10mM Acetate, 10% Glycerol pH 5.5
9. EGF_p55suc9.3: 10mM Acetate, 9.3% Sucrose pH 5.5
10. EGF_p55suc20: 10mM Acetate, 20% Sucrose pH 5.5
11. EGF_p55arg2.5: 10mM Acetate, 2.5% Arginine pH 5.5
12. EGF_p55A5S: 10mM Acetate, 5% Sorbitol pH 5.5
13. EGF_p6gly2.6: 10mM Potassium Phosphate, 2.6% Glycerol pH 6.0
14. EGF_p6gly10: 10mM Potassium Phosphate, 10% Glycerol pH 6.0
15. EGF_p6suc9.3: 10mM Potassium Phosphate, 9.3% Sucrose pH 6.0
16. EGF_p6suc20: 10mM Potassium Phosphate, 20% Sucrose pH 6.0
17. EGF_p6arg2.5: 10mM Potassium Phosphate, 2.5% Arginine pH 6.0
18. EGF_p6A5S: 10mM Potassium Phosphate, 5% Sorbitol pH 6.0

Figure 12 shows the monomer percent of panitumumab analyzed by SE-HPLC. For this characterization, panitumumab was included at 40 mg/mL and stored at -30C for up to one year in the various formulations listed shown below.

| Name | Buffer | Excipients | pH |
|---|---|---|---|
| A5G2.6 | 10 nM Na Acetate | 2.6% Glycerol | 5.0 |
| S5G2.6 | 10mM Succinic acid | 2.6% Glycerol | 5.0 |
| G5G2.6 | 10mM Glutamic acid | 2.6% Glycerol | 5.0 |
| G2.6 | No Buffering Agnet | 2.6% Glycerol | ∼5.8 |
| A57G2.6 | 10mM Acetate | 2.6% Glycerol | 5.7 |
| A58N | 50 mM Na Acetate | 100mM NaCl | 5.8 |

The results indicate that storage at -30C for more than 12 months did not result in any significant differences between any of the above formulations. In this study, the effect of storage in stainless steel containers (S) also was compared with storage in polypropylene (P) bottles as shown in Figure 12. No observable differences between these two containers could be determined.

Figure13 shows the effect of freeze-thaw cycles and storage at -30C on particle formation of panitumumab. The characterized formulations are set forth below. The result indicate acceptable particle numbers for each of the studied formulations.

| Name | Buffer | Excipients | pH |
|---|---|---|---|
| A5G2.6 | 10 nM Na Acetate | 2.6% Glycerol | 5.0 |
| S5G2.6 | 10mM Succinic acid | 2.6% Glycerol | 5.0 |
| G5G2.6 | 10mM Glutamic acid | 2.6% Glycerol | 5.0 |
| G2.6 | No Buffering Agnet | 2.6% Glycerol | ∼5.8 |
| A57G2.6 | 10mM Acetate | 2.6% Glycerol | 5.7 |
| A58N | 50 mM Na Acetate | 100mM NaCl | 5.8 |

Throughout this application various publications have been referenced within parentheses.

## Claims

1. A formulation comprising an acetic acid buffer, a glutamic acid buffer or a succinic acid buffer with a pH between 4.5 - 5.5, at least one excipient comprising a sugar or a polyol, and an effective amount of a therapeutic antibody having specific binding activity to human epidermal growth factor receptor (EGFR), wherein said therapeutic antibody retains at least about 80% stability for up to two months in solution, and wherein said human antibody is panitumumab.

2. The formulation of claim 1, wherein said buffer comprises a sodium salt of acetic acid, glutamic acid or succinic acid.

3. The formulation of claim 1, wherein said acetic acid or acid salt, glutamic acid or acid salt or succinic acid or acid salt is at a concentration of 1-50 mM.

4. The formulation of claim 3, wherein said acetic acid or acid salt, glutamic acid or acid salt, or succinic acid or acid salt is at a concentration of 5-10mM.

5. The formulation of claim 1, wherein said pH is 4.8-5.2.

6. The formulation of claim 1, wherein said sugar or polyol is glycerol, sucrose, trehalose or sorbitol.

7. The formulation of claim 6, wherein said glycerol, sucrose, trehalose or sorbitol is at a concentration of 1-20%.

8. The formulation of claim 6, wherein said glycerol is at a concentration of 2.6%.

9. The formulation of claim 6, wherein said sucrose is at a concentration of 9.3% or 20%.

10. The formulation of claim 6, wherein said trehalose is at a concentration of 9.3% or 20%.

11. The formulation of claim 6, wherein said sorbitol is at a concentration of 5%.

12. The formulation of claim 1, further comprising a surfactant.

13. The formulation of claim 12, wherein said surfactant comprises a polysorbate.

14. The formulation of claim 12, wherein said surfactant is at a concentration of 0.001-0.10 % (w/v).

15. The formulation of claim 1, further comprising a second excipient.

16. The formulation of claim 15, wherein said second excipient is selected from the group consisting of a buffer, stabilizer, tonicity agent, bulking agent, surfactant, cryoprotectant, lyoprotectant, anti-oxidant, metal ion, chelating agent, and preservative.

17. The formulation of claim 1, wherein said therapeutic antibody is at a concentration of 10-200 mg/ml.

18. The formulation of claim 1, wherein said therapeutic antibody is at a concentration of 20 mg/ml.

19. The liquid formulation of claim 1 comprising 10 mM glutamic acid buffer having a pH of 4.8-5.2, at least one excipient comprising about 2.6% glycerol and an effective amount of panitumumab, wherein said panitumumab retains at least about 80% stability for up to two months in solution.

20. The liquid formulation according to claim 19 wherein said formulation further comprises 0.004% polysorbate 80.

## Patentansprüche

1. Formulierung, umfassend einen Essigsäurepuffer, einen Glutaminsäurepuffer oder einen Bernsteinsäurepuffer mit einem pH-Wert zwischen 4,5 - 5,5, mindestens einen Exzipienten, umfassend einen Zucker oder ein Polyol, und eine wirksame Menge eines therapeutischen Antikörpers mit spezifischer Bindungsaktivität an humanen epidermalen Wachstumsfaktorrezeptor (EGFR), wobei der therapeutische Antikörper mindestens etwa 80 % Stabilität für bis zu zwei Monate in Lösung behält und wobei der humane Antikörper Panitumumab ist.

2. Formulierung nach Anspruch 1, wobei der Puffer ein Natriumsalz von Essigsäure, Glutaminsäure oder Bernsteinsäure umfasst.

3. Formulierung nach Anspruch 1, wobei die Essigsäure oder das Säuresalz, die Glutaminsäure oder das Säuresalz oder die Bernsteinsäure oder das Säuresalz in einer Konzentration von 1-50 mM vorliegt.

4. Formulierung nach Anspruch 3, wobei die Essigsäure oder das Säuresalz, die Glutaminsäure oder das Säuresalz oder die Bernsteinsäure oder das Säuresalz in einer Konzentration von 5-10 mM vorliegt.

5. Formulierung nach Anspruch 1, wobei der pH-Wert 4,8-5,2 beträgt.

6. Formulierung nach Anspruch 1, wobei der Zucker oder das Polyol Glycerin, Saccharose, Trehalose oder Sorbit ist.

7. Formulierung nach Anspruch 6, wobei das Glycerin, die Saccharose, die Trehalose oder der Sorbit in einer Konzentration von 1-20 % vorliegt.

8. Formulierung nach Anspruch 6, wobei das Glycerin in einer Konzentration von 2,6 % vorliegt.

9. Formulierung nach Anspruch 6, wobei die Saccharose in einer Konzentration von 9,3 % oder 20 % vorliegt.

10. Formulierung nach Anspruch 6, wobei die Trehalose in einer Konzentration von 9,3 % oder 20 % vorliegt.

11. Formulierung nach Anspruch 6, wobei der Sorbit in einer Konzentration von 5 % vorliegt.

12. Formulierung nach Anspruch 1, weiterhin ein Tensid umfassend.

13. Formulierung nach Anspruch 12, wobei das Tensid ein Polysorbat umfasst.

14. Formulierung nach Anspruch 12, wobei das Tensid in einer Konzentration von 0,001-0,10 % (Gew/Vol) vorliegt.

15. Formulierung nach Anspruch 1, weiterhin einen zweiten Exzipienten umfassend.

16. Formulierung nach Anspruch 15, wobei der zweite Exzipient ausgewählt ist aus der Gruppe bestehend aus einem Puffer, Stabilisator, Tonizitätsmittel, Füllstoff, Tensid, Kälteschutzmittel, Gefrierschutzmittel, Lyoprotektionsmittel, Antioxidationsmittel, Metallion, Chelatbildner und Konservierungsmittel.

17. Formulierung nach Anspruch 1, wobei der therapeutische Antikörper in einer Konzentration von 10-200 mg/ml vorliegt.

18. Formulierung nach Anspruch 1, wobei der therapeutische Antikörper in einer Konzentration von 20 mg/ml vorliegt.

19. Flüssige Formulierung nach Anspruch 1, umfassend 10 mM Glutaminsäurepuffer mit einem pH-Wert von 4,8-5,2, mindestens einen Exzipienten, der etwa 2,6 % Glycerin umfasst, und eine wirksame Menge Panitumumab, wobei das Panitumumab mindestens etwa 80 % Stabilität für bis zu zwei Monate in Lösung behält.

20. Flüssige Formulierung nach Anspruch 19, wobei die Formulierung weiterhin 0,004 % Polysorbat 80 umfasst.

## Revendications

1. Formulation comprenant un tampon acide acétique, un tampon acide glutamique ou un tampon acide succinique avec un pH compris entre 4,5 à 5,5, au moins un excipient comprenant un sucre ou un polyol, et une quantité efficace d'un anticorps thérapeutique ayant une activité spécifique de liaison au récepteur du facteur de croissance épidermique humain (EGFR), ledit anticorps thérapeutique conservant au moins environ 80 % de stabilité jusqu'à deux mois en solution, et ledit anticorps humain étant le panitumumab.

2. Formulation selon la revendication 1, ledit tampon comprenant un sel sodique de l'acide acétique, de l'acide glutamique ou de l'acide succinique.

3. Formulation selon la revendication 1, ledit acide acétique ou sel d'acide, acide glutamique ou sel d'acide ou acide succinique ou sel d'acide se trouvant sous une concentration de 1 à 50 mM.

4. Formulation selon la revendication 3, ledit acide acétique ou sel d'acide, acide glutamique ou sel d'acide, ou acide succinique ou sel d'acide se trouvant sous une concentration de 5 à 10 mM.

5. Formulation selon la revendication 1, ledit pH étant de 4,8 à 5,2.

6. Formulation selon la revendication 1, ledit sucre ou polyol étant le glycérol, le saccharose, le tréhalose ou le sorbitol.

7. Formulation selon la revendication 6, ledit glycérol, saccharose, tréhalose ou sorbitol se trouvant sous une concentration de 1 à 20 %.

8. Formulation selon la revendication 6, ledit glycérol se trouvant sous une concentration de 2,6 %.

9. Formulation selon la revendication 6, ledit saccharose se trouvant sous une concentration de 9,3 % ou 20 %.

10. Formulation selon la revendication 6, ledit tréhalose se trouvant sous une concentration de 9,3 % ou 20 %.

11. Formulation selon la revendication 6, ledit sorbitol se trouvant sous une concentration de 5 %.

12. Formulation selon la revendication 1, comprenant en outre un tensioactif.

13. Formulation selon la revendication 12, ledit tensioactif comprenant un polysorbate.

14. Formulation selon la revendication 12, ledit tensioactif se trouvant sous une concentration de 0,001 à 0,10 % (en pds/vol).

15. Formulation selon la revendication 1, comprenant en outre un second excipient.

16. Formulation selon la revendication 15, ledit second excipient étant sélectionné dans le groupe constitué d'un agent tampon, stabilisant, de tonicité, gonflant, tensioactif, cryoprotecteur, lyoprotecteur, antioxydant, d'un ion métallique, agent chélatant, et d'un agent conservateur.

17. Formulation selon la revendication 1, ledit anticorps thérapeutique se trouvant sous une concentration de 10 à 200 mg/ml.

18. Formulation selon la revendication 1, ledit anticorps thérapeutique se trouvant sous une concentration de 20 mg/ml.

19. Formulation liquide selon la revendication 1 comprenant le tampon acide glutamique 10 mM ayant un pH de 4,8 à 5,2, au moins un excipient comprenant environ 2,6 % de glycérol et une quantité efficace de panitumumab, ledit panitumumab conservant au moins environ 80 % de stabilité jusqu'à deux mois en solution.

20. Formulation liquide selon la revendication 19, ladite formulation comprenant en outre 0,004 % de polysorbate 80.
